# FASCICULE DE BREVET EUROPEEN

(11) **EP 3 288 396 B1**
(45) Date de publication et mention de la délivrance du brevet: **02.10.2019**
(21) Numéro de dépôt: 16724303.9
(22) Date de dépôt: 29.04.2016
(51) Int. Cl.: A23L 2/84, C12H 1/00, C12Q 1/37, C12N 9/50, C12C 1/18, C12C 5/00, C12C 7/14

(54) **UTILISATION DE CYSTEINE ENDOPROTÉASE POUR DIMINUER LE TROUBLE DE BOISSONS**
VERWENDUNG VON CYSTEIN-ENDOPROTEASE ZUR VERRINGERUNG DER TRÜBUNG IN GETRÄNKEN
USE OF CYSTEINE ENDOPROTEASE FOR REDUCING CLOUDINESS IN DRINKS

(30) Priorité: 29.04.2015 FR 1553869
(43) Date de publication de la demande: 07.03.2018
(73) Titulaire: Malteries Soufflet, 10400 Nogent sur Seine (FR); Institut National de la Recherche Agronomique (INRA), 75338 Paris Cedex 07 (FR)
(72) Inventeur: LOPEZ, Michel, 80680 Sains en Amienois (FR); FLISS, Myriam, 10400 La Motte Tilly (FR); MARION, Didier Gilbert, 44300 Nantes (FR)
(74) Mandataire: Lavoix
(86) Numéro de dépôt international: PCT/EP2016/059691
(87) Numéro de publication internationale: WO 2016/174244

(56) Documents cités:
- WO-A1-03/104382
- WO-A1-2014/090803
- WO-A1-2014/191298
- WO-A2-02/46381
- WO-A2-2005/027953

## Description

La présente invention concerne des procédés de clarification de boissons, en particulier de la bière.

La clarification est une étape indispensable dans l'industrie des boissons, confrontée à la formation d'un trouble au cours du stockage de la bière, du vin ou encore des jus de fruits. Etant un problème majeur, de nombreuses études ont été menées sur le sujet et c'est dorénavant un phénomène bien connu. La formation du trouble colloïdal est due aux interactions polyphénols-protéines et protéines-protéines. Le trouble dépend du ratio protéines-polyphénols présent dans le moût mais aussi de la teneur en proline des protéines présentes dans le moût, du degré de polymérisation ainsi que du nombre de groupements hydroxyles des polyphénols (Siebert (2006) LWT 39:987-994)*.*

Plus particulièrement, dans la bière il a été prouvé que les protéines impliquées dans la formation du trouble étaient les hordéines du malt (Steiner et al. (2011) Eur. Food. Res. Technol. 232:191-204). La plupart des hordéines disparaissent au cours des process de brassage, hydrolysées par les protéases endogènes du malt. L'analyse des protéines constituant la bière a toutefois montré la présence d'hordéines B et γ3 résiduelles (Perrocheau et al. (2005) Proteomics 5:2849-2858, Jin et al. (2011) J. Food Biochem. 35:1522-1527) qui sont responsables de la formation du trouble.

Afin de stabiliser la bière, de nombreuses alternatives ont été mises en oeuvre par les brasseurs pour éliminer les composés impliqués dans le trouble. Des moyens physiques peuvent être mis en oeuvre tels que l'élimination des protéines par la bentonite mais cette interaction est aspécifique et élimine les protéines aussi bien impliquées dans la formation du trouble que celles de la mousse. Les mêmes inconvénients sont associés à l'utilisation de l'ultrafiltration.

Il a par ailleurs été proposé d'ajouter des protéines riches en proline, telles que la gélatine, ou des polyphénols, tels que l'acide tannique, dans la boisson avant stockage, de manière à favoriser la précipitation et l'élimination du matériel à l'origine de la formation du trouble. Une autre alternative est d'utiliser des gels de silice qui lient les protéines riches en proline de manière plus spécifique. Ces gels de silice sont toutefois assez peu efficaces pour clarifier les boissons riches en polyphénols. Le polyvinylpolypyrrolidone (PVPP) est souvent utilisé pour extraire la fraction polyphénol. Cependant les polyphénols ont un rôle antioxydant qui peut être bénéfique. La filtration après un passage à basse température induisant la formation du trouble est aussi utilisée mais cette dernière technique a le désavantage d'être longue (plusieurs mois).

Des moyens enzymatiques sont également utilisés tels que l'hydrolyse de la fraction protéique par une protéase naturelle à spécificité d'action très large, la papaïne, mais cette dernière dégrade également les protéines de la mousse ce qui est un effet secondaire indésirable. Plus récemment, l'utilisation d'une proline-spécifique endoprotéase dégradant plus spécifiquement les prolamines a été décrite : le Brewers Clarex® qui est une prolyl-endoprotéase recombinante de la classe EC 3.4.21.26 issue d'un champignon filamenteux: *Aspergillus niger* (US 8,119,171, WO02/046381). Néanmoins, cette endoprotéase peut également dégrader les protéines de la mousse.

Il existe donc toujours un besoin important de moyens alternatifs efficaces pour diminuer le trouble des boissons, en particulier de moyens bons marchés, ne nécessitant pas de modification significative des équipements de fabrication des boissons, et ne présentant pas les inconvénients des techniques connues de l'état de la technique, en particulier n'ayant pas d'impact sur la mousse.

Les grains de céréales, au cours de la germination, produisent des protéases hydrolysant les protéines de réserve pour assurer le développement de la plantule. La présence d'au moins une quarantaine de protéases dans le malt vert a été rapportée par Zhang et Jones (1995) J. Cereal Sci. 21:145-153. Ces protéases appartiennent aux 4 types existants : sérine, métallo, cystéine et aspartyl-protéases. Il a cependant été démontré que les protéases principalement responsables de la dégradation des prolamines pendant la germination des grains sont des cystéines protéases (Shi et Xu (2009) J. Integrative Plant Biol. 51:52-57). Du fait de l'activité de ces protéases sur les prolamines, il a été proposé de les utiliser afin de diminuer la teneur en peptides inducteur de la réponse immunitaire coeliaque dans les produits céréaliers ou au cours de la digestion de ces produits alimentaires (Stenman et al. (2010) Clin. Exp. Immunol. 161:242-249). L'ajout de protéase EP-B d'orge recombinante a par ailleurs récemment été proposé comme additif alimentaire dans l'alimentation des porcs, pour augmenter la digestibilité des protéines d'orge insolubles (Christensen et al. (2014) J. Agric. Food Chem. 62:8562-8570). A la connaissance des inventeurs, aucune de ces applications potentielles n'a été mise en oeuvre et aucune autre application de ces cystéine endoprotéases n'a été suggérée jusqu'à présent.

La présente invention résulte de la découverte inattendue par les inventeurs que le malt, en particulier un extrait de malt, contient une activité protéasique capable de diminuer efficacement le trouble de la bière. Les inventeurs ont plus particulièrement démontré que cette activité protéasique implique des cystéine endoprotéases, et plus particulièrement, dans le malt d'orge, les endoprotéases A et B.

L'utilité des endoprotéases A et B d'orge est d'autant plus surprenante que, contrairement à de nombreuses protéases, la présence de proline, acide aminé largement présent dans les hordéines, ne semble pas pénaliser leur activité protéolytique. Les cystéine-endoprotéases des céréales sont capables de cliver les domaines répétés N-terminaux des hordéines et gliadines, riches en proline et glutamine (Bethune et al. (2006) Chem. Biol. 13:637-647).

Par ailleurs, les inventeurs ont démontré que cette activité protéasique permettait en parallèle, de manière surprenante d'augmenter le rendement de fermentation lors de la fabrication de la bière.

La présente invention concerne donc l'utilisation d'au moins une cystéine endoprotéase comprenant une séquence au moins 55% identique à la séquence SEQ ID NO : 1, à la séquence SEQ ID NO : 2, à la séquence SEQ ID NO : 3 et/ou à la séquence SEQ ID NO : 4, ou un fragment de celle-ci présentant une activité cystéine endoprotéase, pour diminuer le trouble d'une boisson, fermentée ou non, à base de céréales.

La présente invention a également pour objet l'utilisation d'un extrait de malt comprenant une activité endoprotéasique pour diminuer le trouble d'une boisson, fermentée ou non, à base de céréales.

Un autre objet de l'invention concerne l'utilisation (i) d'au moins une cystéine endoprotéase telle que définie ci-dessus ou (ii) d'un extrait de malt tel que défini ci-dessus, dans la préparation d'une boisson, fermentée ou non, à base de céréales.

La présente invention concerne également l'utilisation (i) d'au moins une cystéine endoprotéase telle que définie ci-dessus ou (ii) d'un extrait de malt tel que défini ci-dessus, pour augmenter le rendement de fermentation lors de la fabrication de boissons fermentées, à base de céréales.

La présente description décrit également un procédé de fabrication d'une boisson, fermentée ou non, à base de céréales, comprenant une étape d'ajout, dans le moût ou le jus, (i) d'au moins une cystéine endoprotéase telle que définie ci-dessus ou (ii) d'un extrait de malt tel que défini ci-dessus.

Un autre objet de l'invention concerne une boisson à base de céréales susceptible d'être obtenue par l'un des procédés selon l'invention.

### Description détaillée de l'invention

### Boisson à base de céréales

Par "boisson", on entend ici les boissons à tous les stades de leur préparation. Par conséquent, une boisson n'est pas seulement une boisson prête à être consommée mais également n'importe quelle composition utilisée pour préparer la boisson. De préférence, une boisson dans le contexte de l'invention est une boisson prête à être consommée.

Par "boisson à base de céréales", on entend ici toute boisson dont au moins l'une des matières premières utilisées pour sa préparation provient d'une céréale. La boisson à base de céréales obtenue dans le contexte de l'invention peut ainsi être une boisson à base d'orge, de blé, en particulier de froment, d'avoine, de seigle, de maïs, de riz, de sorgho, de millet, de pseudo-céréales telles que le quinoa ou le sarrasin, ou de mélanges de ceux-ci. De préférence, la boisson à base de céréales obtenue dans le contexte de l'invention est une boisson à base d'orge ou de blé.

La boisson à base de céréales obtenue dans le cadre de l'invention peut être une boisson fermentée ou non fermentée. De préférence, il s'agit d'une boisson fermentée.

Le trouble observé lors du stockage des boissons, fermentées ou non, à base de céréales, est généralement dû aux interactions polyphénols-protéines et/ou protéines-protéines. Par conséquent, dans un mode de réalisation particulier, la boisson, fermentée ou non, à base de céréales obtenue dans le cadre de l'invention comprend des protéines et des polyphénols.

Les inventeurs ont montré que les cystéine endoprotéases et/ou l'extrait de malt étaient particulièrement efficaces pour prévenir ou diminuer le trouble de la bière.

Par conséquent, dans un mode de réalisation particulier, la boisson, fermentée ou non, à base de céréales est un liquide utilisé dans la production de la bière. De manière particulièrement préférée, la boisson, fermentée ou non, à base de céréales est une bière.

Par "bière", on entend ici les bières obtenues à partir de maisches préparées à partir de céréales non-maltées, à partir de maisches préparées à partir de céréales maltées, ou à partir de maisches préparées à partir d'un mélange de céréales maltées et non maltées. Le terme "bière" couvre ici les bières de fermentation basse, c'est-à-dire dont la fermentation est mise en oeuvre à une température généralement comprise entre 8 et 12°C, les bières de fermentation haute, c'est-à-dire dont la fermentation est mise en oeuvre à une température généralement comprise entre 20 et 25°C, les bières de fermentation simple, double ou triple (dont les températures de fermentation sont modifiées en cours de fermentation), les bières à fermentation spontanée, c'est-à-dire dont la fermentation ne nécessite pas d'ajout de levure dans le moût, les bières à fermentation mixte, c'est-à-dire dont la fermentation combine la fermentation avec une levure et une bactérie (de type lactique ou acidifiante) ou avec deux levures, les bières préparées avec des additifs et les bières avec une gamme de taux d'alcool de 0 à 10%.

### Diminution du trouble

Les termes "trouble" et "turbidité" sont ici utilisés de manière interchangeable. Le trouble peut être mesuré par toute technique bien connue de l'homme du métier, typiquement au moyen d'un turbidimètre, par exemple inclus dans un Tannometer. Brièvement, dans un turbidimètre, la lumière rayonnée est réfléchie par les particules (turbidité) existantes. La lumière diffusée est ensuite mesurée par un photodétecteur disposé en angle droit (90°C) par rapport à la source lumineuse. La turbidité est exprimée en UTN (Unité de Turbidité Néphélométrique, NTU en anglais) ou en EBC (European Brewery Convention), 1 UTN équivalant à 0,25 EBC. Elle est évaluée de préférence à une température de -8°C. La turbidité est de préférence mesurée au moyen du test Chapon décrit dans Chapon et al. (1993) J. Inst. Brew. 99:49-56, en particulier le test "Alcohol-Chill-Test" décrit dans Chapon et al. (1993) J. Inst. Brew. 99:49-56. Typiquement, 9,4 ml d'échantillon sont mélangés avec 0,6 ml d'éthanol. Après incubation de préférence pendant au moins 30 min, par exemple pendant 30 min ou 40 min, à -8°C, la turbidité est mesurée à -8°C sur un turbidimètre, typiquement sur un Tannometer, tel que commercialisé par la société Pfeuffer. Les résultats sont alors typiquement exprimés en unités EBC.

Les termes "diminution du trouble", "réduction de la turbidité" et "clarification" sont ici utilisés de manière interchangeable. De préférence, les cystéines endoprotéases et l'extrait de malt utilisés dans le cadre de l'invention permettent de réduire le trouble de la boisson de manière significative par rapport à la même boisson non traitée.

De préférence, les cystéines endoprotéases et l'extrait de malt utilisés dans le cadre de l'invention permettent de réduire la turbidité de la boisson d'au moins 70% par rapport à la même boisson non traitée, de manière davantage préférée d'au moins 71%, au moins 73%, au moins 74%, au moins 75%, au moins 77%, au moins 80%, au moins 85%, au moins 86%, ou de manière encore préférée d'au moins 93%, au moins 94% ou au moins 95%, en particulier lorsque la turbidité est mesurée à -8°C, plus particulièrement au moyen du test Chapon tel que décrit ci-dessus.

De préférence, les cystéines endoprotéases et l'extrait de malt utilisés dans le cadre de l'invention permettent de réduire le trouble de la boisson d'au moins 65 unités EBC par rapport à la même boisson non traitée, de manière davantage préférée d'au moins 67 unités EBC, au moins 68 unités EBC, au moins 70 unités EBC, au moins 74 unités EBC, au moins 75 unités EBC, au moins 80 unités EBC, au moins 81 unités EBC, au moins 87 unités EBC, au moins 89 unités EBC, au moins 90 unités EBC, au moins 94 unités EBC, au moins 95 unités EBC, au moins 100 unités EBC, au moins 102 unités EBC ou au moins 103 unités EBC, en particulier lorsque la turbidité est mesurée à -8°C, plus particulièrement au moyen du test Chapon tel que décrit ci-dessus.

### Augmentation du rendement de fermentation

Par « augmentation du rendement de fermentation », on entend ici qu'à durée équivalente de fermentation, il reste moins de sucres fermentescibles encore présents dans le moût par rapport à différents témoins, en particulier positifs et/ou négatifs. Les témoins négatifs, de préférence deux témoins négatifs, peuvent être constitués par un moût sans aucun ajout et un moût avec ajout d'extrait brut de malt d'orge dénaturé par la chaleur, et le témoin positif peut être constitué par un moût avec ajout de Brewers Clarex®.
Le suivi de la fermentation est typiquement effectué par suivi de la population de levure, et du pourcentage en masse d'extrait sec du moût, exprimé en degrés Plato (noté °Plato). 1°Plato correspond à 1 g de matière sèche soluble (essentiellement les sucres fermentescibles) pour 100 g de moût. La quantité de sucre comprise initialement dans le moût détermine la quantité d'alcool et de dioxyde de carbone dans la bière produite. Le degré Plato se mesure typiquement au moyen d'un densimètre automatique (densimètre Anton-Paar DMA35).

Les inventeurs ont ainsi montré que les cystéines endoprotéases et l'extrait de malt utilisés dans le cadre de l'invention permettent d'atteindre un °Plato d'au plus 1 à J0+8, lorsqu'ils étaient ajoutés au cours de l'étape de fermentation.

Ainsi, de manière préférée, les cystéines endoprotéases ou l'extrait de malt selon l'invention augmentent le rendement de fermentation de manière à atteindre un °Plato 8 jours après le début de la fermentation d'au plus 1, de manière davantage préférée d'au plus 0,8, au plus 0.7, au plus 0.6, au plus 0.5 ou au plus 0.2 °Plato, en particulier lorsque la fermentation est effectuée à une température d'environ 13°C et en particulier lorsqu'un moût à 11° Plato est produit par brassage de type « congress EBC».

### Cystéine endoprotéase

Par "cystéine endoprotéase" ou "cystéine endopeptidase", on entend ici une enzyme de la classe EC 3.4.22 dont le résidu d'acide aminé nucléophile de la triade catalytique est une cystéine et clivant les protéines à l'intérieur de la chaîne peptidique.

La ou les cystéine endoprotéase(s) utilisée(s) dans le cadre de l'invention comprend, ou consiste, en une séquence au moins 55% identique, de manière davantage préférée au moins 60% identique, au moins 65% identique, au moins 70% identique, au moins 75% identique, au moins 80% identique, au moins 85% identique, au moins 90% identique, au moins 91, 92, 93, 94, 95, 96, 97, 98, 99 ou 100 % identique à la séquence SEQ ID NO : 1, à la séquence SEQ ID NO : 2, à la séquence SEQ ID NO : 3 et/ou à la séquence SEQ ID NO : 4, ou un fragment de celle-ci présentant une activité cystéine endoprotéase.

Par "séquence au moins x% identique à une séquence de référence", on entend ici que la séquence est identique à la séquence de référence ou diffère de la séquence de référence par jusqu'à 100-x altérations d'acides aminés pour chaque 100 acides aminés de la séquence de référence. Les altérations d'acides aminés par rapport à la séquence de référence peuvent être des substitutions, délétions et/ou insertions d'un ou de plusieurs acides aminés, et ceci à des positions telles que ces modifications ne portent pas significativement atteinte à l'activité enzymatique des enzymes. Les substitutions peuvent notamment correspondre à des substitutions conservatives ou à des substitutions d'acides aminés naturels par des acides aminés non naturels ou pseudo-acides aminés. Dans un mode de réalisation particulier, la séquence protéique ne diffère de la séquence de référence que par la présence de substitutions conservatives. Les substitutions conservatives sont des substitutions d'acides aminés de même classe, telles que des substitutions d'acides aminés aux chaînes latérales non chargées (tels que l'asparagine, la glutamine, la serine, la cystéine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique), d'acides aminés aux chaînes latérales apolaires (tels que l'alanine, la valine, la leucine, l'isoleucine, la proline, la phénylalanine, la méthionine et le tryptophane).

L'alignement et la détermination du pourcentage d'identité peut être effectué manuellement ou automatiquement en utilisant par exemple le programme Needle qui est basé sur l'algorithme de Needleman et Wunsch, décrit dans Needleman et Wunsch (1970) J. Mol Biol. 48:443-453, avec par exemple les paramètres suivants pour la comparaison de séquences protéiques : matrice de comparaison : BLOSUM62, gap open penalty = 10, gap extend penalty = 0.5, end gap penalty : false, end gap open penalty = 10, end gap extend penalty = 0.5; et les paramètres suivants pour la comparaison de séquences nucléiques : matrice de comparaison : DNAFULL; gap open penalty = 10, gap extend penalty = 0.5, end gap penalty : false, end gap open penalty = 10, end gap extend penalty = 0.5.

Les séquences SEQ ID NO: 1 et SEQ ID NO: 2 correspondent aux séquences d'acides aminés de cystéine endoprotéases EP-A d'orge.

La cystéine endoprotéase EP-A d'orge est décrite dans Koehler et Ho (1988) Plant Physiol. 87:95-103. EP-A a un poids moléculaire apparent de 37 kDa, et présente une activité optimale à pH de 5,0 et une température de 45°C. Elle est induite par l'acide gibbérellique. Son site de coupure sur des hordéines de type C recombinantes a été caractérisé dans Davy et al. (1998) Plant Physiol. 117:255-261 : EP-A coupe les hordéines après les acides aminés arginine et glutamine, avec une préférence pour l'arginine. La présence, au niveau du site de clivage, d'un acide aminé tel que la phénylalanine, la leucine ou la valine avant l'arginine ou la glutamine, favorise l'hydrolyse tandis que la présence de proline avant ou après ces deux résidus diminue significativement son efficacité catalytique mais ne l'inhibe pas. Les spécificités catalytiques d'EP-A sur les prolamines restent peu caractérisées, même s'il est vraisemblable, sur la base de l'identité de séquence d'EP-A et d'EP-B (52% pour les préproprotéines et 60% pour la protéine maturée) et une conservation stricte des acides aminés du site actif, impliqués dans la catalyse et dans l'ancrage du segment peptidique selon la structure cristallographique du complexe EP_B-leupeptine décrite par Bethune *et al.* (2006), que EP-A et EP-B présentent des spécificités catalytiques identiques. Deux variants de la cystéine endoprotéase EP-A d'orge ont été identifiés: il s'agit des variants de séquences : et

Différents domaines ont été identifiés dans les séquences protéiques d'EP-A. Ainsi, les acides aminés 1 à 23 de SEQ ID NO: 1 ou SEQ ID NO: 2 correspondent au peptide signal et les acides aminés 128 à 365 de SEQ ID NO: 1 ou SEQ ID NO: 2 correspondent à la chaine mature (séquences SEQ ID NO: 5 et 6). Le site catalytique d'EP-A n'a pas été caractérisé. Le site catalytique peut toutefois être déduit par homologie avec EP-B comme contenant les acides aminés suivants: cystéine 29, histidine 164, sérine 28, glycine 71, aspartate 163, asparagine 185, glutamine 23 numérotés sur la base de la séquence des protéines matures de séquence SEQ ID NO: 5 ou SEQ ID NO: 6.

Les séquences SEQ ID NO: 3 et SEQ ID NO: 4 correspondent aux séquences d'acides aminés de cystéine endoprotéases EP-B d'orge.

La cystéine endoprotéase EP-B d'orge est décrite dans Koehler et Ho (1990) Plant Physiol. 94:251-258. EP-B a un poids moléculaire apparent de 30 kDa (Davy et al. (1998) Plant Physiol. 117:255-261), et présente une activité optimale à un pH de 4,5 et une température de 40°C pour des substrats protéiques modèles (azocaséine ou hémoglobine). Dans ces conditions optimales, les hordéines sont typiquement clivées en de multiples fragments de masse moléculaire comprise entre 2000 et 25000 Daltons. EP-B est induite par l'acide gibbérellique. Son site de coupure sur des hordéines de type C recombinantes a été caractérisé dans Davy et al. (1998) Plant Physiol. 117:255-261 : EP-B coupe les hordéines après les acides aminés arginine et glutamine, avec une préférence pour l'arginine pour les coupures primaires. La présence, au niveau du site de clivage, d'un acide aminé tel que la phénylalanine, la leucine ou la valine avant l'arginine ou la glutamine, favorise l'hydrolyse. Contrairement à EP-A, EP-B conserve une bonne activité catalytique malgré la présence de proline après ces deux résidus arginine ou glutamine. Des sites secondaires de clivage peuvent être mis en évidence quand l'hydrolyse de la C-hordéine par l'EP-B est prolongée. L'endoprotéase peut cliver après une glutamine, une arginine, une tyrosine, une glycine, un acide glutamique, une sérine, une histidine, une leucine. Elle ne coupe apparemment pas en N- ou C-terminal d'une proline dans le cas de la C-hordéine (Davy *et al*., 1998) et ne peut donc être assimilée à une endoprolidase. Des résultats similaires ont été obtenus pour l'hydrolyse d'une a2-gliadine par l'EP-B recombinante et montre une nette préférence pour des coupures en C-terminal de glutamine (Bethune *et al*., 2006). La spécificité de clivage des cystéines endoprotéases de céréales est par ailleurs très différente de celle des autres cystéine endoprotéases comme la papaïne qui est utilisée pour diminuer le trouble de la bière. La papaïne est en effet relativement peu spécifique (Kimmel et Smith (1954) J. Biol. Chem 207:515-531).

Deux variants de la cystéine endoprotéase EP-B d'orge ont été identifiés: il s'agit des variants de séquences : et

Différents domaines ont été identifiés dans les séquences protéiques d'EP-B. Ainsi, les acides aminés 1 à 28 de SEQ ID NO: 3 ou SEQ ID NO: 4 correspondent au peptide signal, les acides aminés 29 à 130 de SEQ ID NO: 3 ou SEQ ID NO: 4 correspondent au propeptide d'activation et les acides aminés 131 à 371 de SEQ ID NO: 3 (séquence SEQ ID NO: 7) ou 131 à 373 de SEQ ID NO: 4 (séquence SEQ ID NO: 8) correspondent à la chaine mature. Le site catalytique d'EP-B a en outre été caractérisé. Il implique principalement la cystéine 158 (cystéine 28 pour la protéine mature de séquence SEQ ID NO: 7 ou 8) et l'histidine 297 (histidine 167 pour la protéine mature de séquence SEQ ID NO: 7 ou 8) de SEQ ID NO: 3 ou SEQ ID NO: 4.

Dans ces deux protéines, l'aspartate 296 (aspartate 166 dans la protéine mature) intervient dans le positionnement du substrat par rapport à la cystéine 158 (cystéine 28 dans la protéine mature) catalytique (Bethune *et al*., 2006).

La cystéine endoprotéase utilisée dans le cadre de l'invention peut avoir une activité cystéine endoprotéase A ou cystéine endoprotéase B.

De manière particulièrement préférée, la ou les cystéine endoprotéase(s) utilisée(s) dans le cadre de l'invention est (i) une cystéine endoprotéase A comprenant, ou consistant en, une séquence au moins 55% identique, ou au moins 60, 65, 70, 75, 80, 85, 90, 91, 92, 93, 94, 95, 96, 97, 98, 99 ou 100% identique, à la séquence SEQ ID NO: 1 et/ou à la séquence SEQ ID NO : 2, et/ou un fragment de celle-ci présentant une activité cystéine endoprotéase A, et/ou (ii) une cystéine endoprotéase B comprenant, ou consistant en, une séquence au moins 55% identique, ou au moins 60, 65, 70, 75, 80, 85, 90, 91, 92, 93, 94, 95, 96, 97, 98, 99 ou 100% identique, à la séquence SEQ ID NO: 3 et/ou à la séquence SEQ ID NO : 4, et/ou un fragment de celle-ci présentant une activité cystéine endoprotéase B.

De manière préférée entre toutes, la ou les cystéine endoprotéase(s) utilisée(s) dans le cadre de l'invention est (i) la cystéine endoprotéase EP-A d'orge comprenant, ou consistant en, la séquence SEQ ID NO: 1 ou SEQ ID NO: 2, et/ou (ii) la cystéine endoprotéase EP-B d'orge comprenant, ou consistant en, la séquence SEQ ID NO: 3 ou SEQ ID NO: 4.

Par "fragment" d'une séquence de référence, on entend ici une séquence qui a une taille inférieure à la séquence de référence. Dans le cadre de la présente invention, les fragments peuvent par exemple avoir une taille comprise entre 230 et 373 acides aminés, de 235 à 371 acides aminés, de 236 à 365 acides aminés, de 237 à 350 acides aminés, de 239 à 300 acides aminés, de 240 à 290 acides aminés, de 250 à 280 acides aminés ou de 260 à 270 acides aminés. De préférence, dans le cadre de l'invention, les fragments contiennent le site actif de l'enzyme dont ils sont dérivés. Les fragments utilisés dans le cadre de l'invention peuvent être obtenus par clivage enzymatique ou non enzymatique de l'enzyme mature ou de la proprotéine dont ils sont dérivés, conservant ou amplifiant la spécificité et l'efficacité endoprotéasique.

Les fragments utilisés dans le cadre de la présente invention présentent une activité cystéine endoprotéasique. Des fragments des séquences protéiques définies ci-dessus présentant une activité cystéine endoprotéasique peuvent être identifiés par l'homme du métier par des techniques de routine. En effet, comme il est bien connu de l'homme du métier, les cystéine endoprotéase comprennent un signal peptide et l'activité cystéine endoprotéasique est donc située au niveau de la chaine mature la cystéine endoprotéase.

Ainsi, dans un mode de réalisation particulièrement préféré, le fragment de cystéine endoprotéase utilisé dans le cadre de l'invention comprend, ou consiste, en une séquence au moins 55% identique, ou au moins 60, 65, 70, 75, 80, 85, 90, 91, 92, 93, 94, 95, 96, 97, 98, 99 ou 100% identique à la séquence SEQ ID NO: 5, SEQ ID NO: 6, SEQ ID NO: 7 et/ou SEQ ID NO: 8, , ledit fragment présentant une activité cystéine endoprotéasique.

Par ailleurs, comme il est bien connu de l'homme du métier le site catalytique des cystéine endoprotéasique est situé au sein d'une poche contenant la cystéine et une histidine impliquées dans la catalyse et différents acides aminés impliqués dans la fixation et/ou la reconnaissance du site de coupure comme décrit ci-dessus pour EP-A. Des fragments des séquences protéiques définies ci-dessus présentant une activité cystéine endoprotéasique comprendront donc de préférence, ou consisteront de préférence en, des résidus d'acides aminés du site catalytique des cystéines endoprotéases définies ci-dessus.

Ainsi, dans un mode de réalisation préféré, le fragment de cystéine endoprotéase utilisé dans le cadre de l'invention comprend, ou consiste, en un fragment de la préprotéine ou de la protéine mature ou tout fragment protéique fonctionnel contenant les résidus d'acides aminés du site catalytique décrit précédemment.

L'homme du métier pourra ensuite vérifier que ces fragments présentent une activité cystéine endoprotéasique en mesurant leur activité par des techniques bien connues de l'état de la technique, par exemple avec l'utilisation d'inhibiteurs spécifiques des cystéine endoprotéases, tel que l'E64 (Trans-epoxysuccinyl-L-leucocylamido-(-4-guanidino)butane).

Les techniques de mesure d'une activité cystéine endoprotéasique sont en effet bien connues de l'homme du métier. Typiquement, elles comprennent des techniques colorimétriques ou fluorimétriques utilisant des di- ou tripeptides avec un chromophore ou un fluorophore, des techniques utilisant des protéines de réserve des céréales (prolamines) ou des fragments de ces protéines couplées à une analyse des peptides produits par spectrométrie de masse. Typiquement, l'activité cystéine endoprotéasique peut être mesurée en utilisant un peptide fluorescent comme le N-CBZ (benzyloxycarbonyl)-Phe (phénylalanine)-Arg (arginine)- AMC (7-amido-4-methyl-coumarine) dans un tampon citrate 50 mM (pH 4 contenant 2 mM cystéine et 2 mM de β-mercaptoéthanol). Le substrat, N-CBZ-Phe-Arg-AMC, est solubilisé dans le DMSO à une concentration de 100 µM et 10 µl sont ajoutés à une cuvette en quartz contenant 2 mL du tampon citrate. Entre 10 et 30 µl de solution enzymatique sont typiquement ajoutées et l'augmentation de fluorescence est mesurée au cours du temps (excitation 360 nm, émission 460 nm) à la température de 20°C. La vitesse initiale de la réaction est déduite de la mesure de pente de la courbe de l'intensité de fluorescence en fonction du temps. La fluorescence totale est mesurée en utilisant une solution de papaïne à 0.2 µM.

La au moins une cysteine endoprotéase utilisée dans le cadre de l'invention peut être utilisée sous forme isolée ou purifiée.

Par "isolé" ou "purifié", on entend ici une cystéine endoprotéase prélevée de son environnement naturel. Par exemple, une cystéine endoprotéase produite de manière recombinante dans des cellules hôtes est considérée comme isolée dans le contexte de l'invention, tout comme un polypeptide recombinant ou natif qui a été purifié substantiellement par n'importe quelle technique appropriée.

La au moins une cystéine endoprotéase utilisée dans le cadre de l'invention peut être sous forme isolée. Elle peut toutefois être mélangée dans des véhicules ou diluants qui n'interféreront pas avec l'effet recherché de cette enzyme, et sera alors toujours considérée comme étant isolée.

La au moins une cystéine endoprotéase utilisée dans le cadre de l'invention peut également être sous forme plus substantiellement purifiée, qui inclura la au moins une cystéine endoprotéase dans une préparation dans laquelle plus de 70%, ou plus de 80%, 90%, 95%, 98% ou 99% des protéines dans la préparation est la au moins une cystéine endoprotéase.

La cystéine endoprotéase utilisée dans le cadre de l'invention peut être sous forme d'un produit naturel brut ou purifié, un produit obtenu par synthèse chimique, un produit obtenu par une technique recombinante à partir d'un hôte eucaryote ou procaryote, tel qu'une cellule bactérienne, de levure, fongique, de plante supérieure, d'insecte ou de mammifère.

Dans un procédé de production par une technique recombinante, un vecteur contenant un acide nucléique codant pour une cystéine endoprotéase est de préférence transféré dans une cellule hôte qui est mise en culture dans des conditions permettant l'expression de la protéine correspondante. L'enzyme produite peut ensuite être récupérée et purifiée. La séquence d'acide nucléique d'intérêt peut être insérée dans un vecteur d'expression, dans lequel elle est liée de manière opérante à un ou des élément(s) permettant son expression ou la régulation de son expression, tels que notamment des promoteurs, activateurs et/ou terminateurs de transcription. Les signaux contrôlant l'expression des séquences nucléotidiques (promoteurs, activateurs, séquences de terminaison...) sont choisis en fonction de l'hôte cellulaire utilisé. A cet effet, les séquences nucléotidiques codant la protéine d'intérêt peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation ou la précipitation au phosphate de calcium. Des exemples de cellules hôtes incluent notamment des cellules humaines telles que HEK293, PER.C6, des cellules de mammifères non humains telles que CHO, COS, MDCK, des cellules d'insectes telles que les cellules SF9, des bactéries telles que *E. coli*, des souches de champignon et/ou de levures telles que L40 et Y90.

Dans un mode de réalisation particulier, la au moins une cystéine endoprotéase utilisée dans le cadre de l'invention peut être obtenue à partir d'extraits de végétaux dans lesquels elle est naturellement produite.

La au moins une cystéine endoprotéase utilisée dans le cadre de l'invention peut ainsi être obtenue à partir de protoplastes de cellules aleuroniques ou d'une culture cellulaire de la couche aleurone. Les techniques de culture des protoplastes des cellules aleuroniques ou de la couche à aleurone sont bien connues de l'homme du métier et décrites par exemple dans Chrispeels & Varner (1967) Plant Physiol 42:398-406 et Taiz & Jones (1971) Planta 101:95-100**.** Les cystéine endoprotéases étant induites par l'acide gibbérellique, dans un mode de réalisation particulier, les cellules de la couche aleurone sont cultivées en présence d'acide gibbérellique, de préférence à une concentration d'environ 1 µM.

La germination des grains de céréales induisant l'expression de cystéine endoprotéases, la au moins une cystéine endoprotéase utilisée dans le cadre de l'invention peut également être obtenue à partir de grains de céréales germés, en particulier à partir de malt de céréale, tel que décrit dans la section "*Extrait de malt*" ci-dessous.

Une cystéine endoprotéase utilisée dans le contexte de l'invention peut être obtenue à partir de cultures de cellules recombinantes, à partir de cultures de cellules végétales ou à partir de végétaux ou de parties de végétaux, en particulier à partir de lysats et extraits de cellules, de végétaux ou de parties de végétaux, ou du surnageant du milieu de culture, par des techniques bien connues de l'homme du métier, utilisées individuellement ou en combinaison, telles que la précipitation au sulfate d'ammonium ou à l'éthanol, l'extraction acide, des méthodes chromatographiques, des techniques d'immunoaffinité à l'aide d'anticorps mono- ou polyclonaux spécifiques, etc.

Elle peut également être obtenue sous forme brute, par simple extraction à partir de cultures de cellules recombinantes, à partir de cultures de cellules végétales ou à partir de végétaux ou de parties de végétaux, ou du surnageant du milieu de culture.

Ainsi dans un mode de réalisation particulier, la au moins une cystéine endoprotéase est utilisée sous forme d'extrait de malt tel que défini à la section "*Extrait de malt*" ci-dessous.

### Extrait de malt

Par "malt", on entend ici une céréale germée obtenue par un procédé de maltage.

Le malt peut être obtenu par toute technique de maltage bien connue de l'homme du métier. Le maltage se déroule typiquement en 4 étapes incluant le trempage qui permet d'humidifier le grain, la germination lors de laquelle le grain commence à germer et qui donne naissance au "malt vert", le touraillage lors duquel le malt vert est séché pour obtenir un malt sec, et le dégermage lors duquel le malt est débarrassé de ses radicelles. De préférence, le malt utilisé dans le cadre de l'invention est un malt sec. De manière particulièrement préférée, le malt utilisé dans le cadre de l'invention est un malt sec obtenu après 5 à 11 jours de germination.

Le malt utilisé dans le cadre de l'invention est de préférence un malt de céréale, en particulier un malt d'orge, de seigle ou de blé, de manière plus particulièrement préférée un malt d'orge, plus particulièrement un malt d'orge d'hiver ou un malt d'orge de printemps.

Le malt utilisé dans le cadre de l'invention peut être un malt diastasique ou non-diastasique.

Par "extrait de malt", on entend ici une préparation obtenue suite à l'extraction d'un malt avec un solvant approprié tel que, par exemple, de l'eau, des tampons aqueux, de l'éthanol, un mélange de ceux-ci, des huiles ou d'autres tampons aqueux appropriés bien connus dans le domaine des extractions végétales. L'extrait de malt utilisé dans le cadre de l'invention peut en particulier être un extrait de malt, de préférence de malt sec, éventuellement concentré.

L'extrait de malt utilisé dans le cadre de l'invention peut être sous forme lyophilisée et éventuellement remis en suspension avant utilisation.

L'extrait de malt utilisé dans le cadre de l'invention comprend une activité endoprotéasique.

Par "activité endoprotéasique", on entend ici une activité enzymatique protéasique capable de cliver une protéine ou un peptide à l'intérieur de la chaine peptidique. Les techniques pour mettre en évidence une activité endoprotéasique sont bien connues de l'homme du métier et décrites dans la section "*Cystéine endoprotéase*" ci-dessus.

De préférence, l'extrait de malt comprenant une activité endoprotéasique utilisé dans le cadre de l'invention entraine une diminution de la turbidité d'une boisson, fermentée ou non, à base de céréales, telle que définie dans la section "*Boisson à base de céréales*" ci-dessus, par rapport à une boisson non traitée, d'au moins 70%, de manière davantage préférée d'au moins 75%, au moins 80%, au moins 85%, au moins 90%, ou de manière encore préférée d'au moins 95%, en particulier lorsque la turbidité est mesurée à -8°C, plus particulièrement au moyen du test Chapon tel que décrit dans la section "*Diminution du trouble*" ci-dessus.

De préférence, l'extrait de malt comprenant une activité endoprotéasique utilisé dans le cadre de l'invention entraine une diminution de la turbidité d'une boisson, fermentée ou non, à base de céréales, telle que définie dans la section "*Boisson à base de céréales*" ci-dessus, par rapport à une boisson non traitée, d'au moins 65 unités EBC, de manière davantage préférée d'au moins 70 unités EBC, au moins 75 unités EBC, au moins 80 unités EBC, au moins 85 unités EBC, au moins 90 unités EBC, au moins 95 unités EBC, ou au moins 100 unités EBC, en particulier lorsque la turbidité est mesurée à -8°C, plus particulièrement au moyen du test Chapon tel que décrit dans la section "*Diminution du trouble*" ci-dessus.

Un extrait de malt présentant de telles caractéristiques peut être typiquement un extrait de malt brut obtenu par récupération du surnageant de centrifugation d'un malt, tel que défini ci-dessus, broyé mis en suspension dans une solution aqueuse.

De manière davantage préférée, un extrait de malt présentant de telles caractéristiques est un extrait de malt traité obtenu par récupération et filtration de centrifugation d'un extrait de malt brut tel que défini ci-dessus, traité par précipitation différentielle au sulfate d'ammonium, en particulier traité entre 15% et 20%, de préférence à 26%, de saturation au sulfate d'ammonium, puis éventuellement à au moins 50%, de préférence à 89%, de saturation au sulfate d'ammonium, ou par tout pourcentage de saturation au sulfate d'ammonium bien connu de l'homme du métier.

Comme montré dans les exemples, les inventeurs ont en effet démontré qu'un extrait de malt obtenu par l'une de ces techniques permettait de diminuer le trouble d'une boisson, fermentée ou non, à base de céréales, dans une proportion conforme à celle mentionnée ci-dessus. L'extrait de malt utilisé dans le cadre de l'invention peut toutefois être obtenu par toute technique comprenant des étapes supplémentaires d'extraction et/ou de purification bien connues de l'homme du métier, telles que des techniques de chromatographies échangeuses d'ions, de chromatographie d'exclusion de taille ou de chromatographie d'interaction hydrophobe.

Les inventeurs ont par ailleurs montré qu'un extrait de malt obtenu par l'une de ces techniques comprenait une activité cystéine endoprotéasique.

De préférence, l'extrait de malt utilisé dans le cadre de l'invention comprend donc une activité cystéine endoprotéasique, comme définie à la section "*Cystéine endoprotéase*" ci-dessus. De manière davantage préférée, l'extrait de malt utilisé dans le cadre de l'invention comprend une activité cystéine endoprotéase A et/ou cystéine endoprotéase B, telle que définie à la section "*Cystéine endoprotéase*" ci-dessus.

Les inventeurs ont également montré qu'un extrait de malt obtenu par l'une de ces techniques permettait d'augmenter le rendement de fermentation lors de la fabrication de boissons fermentées, à base de céréales.

### Procédé de fabrication de boisson

La présente invention concerne l'utilisation (i) d'au moins une cystéine endoprotéase telle que définie dans la section "*Cystéine endoprotéase*" ci-dessus ou (ii) d'un extrait de malt tel que défini dans la section "*Extrait de malt*" ci-dessus, dans la préparation d'une boisson, fermentée ou non, à base de céréales, telle que définie dans la section "*Boisson à base de céréales*" ci-dessus.

La présente invention a également pour objet un procédé de fabrication d'une boisson, fermentée ou non, à base de céréales, telle que définie dans la section "*Boisson* à *base de céréales*" ci-dessus, comprenant une étape d'ajout (i) d'au moins une cystéine endoprotéase telle que définie dans la section "*Cystéine endoprotéase*" ci-dessus ou (ii) d'un extrait de malt tel que défini dans la section "*Extrait de malt*" ci-dessus, en particulier dans le jus ou le moût.

Les étapes d'un procédé de fabrication d'une boisson, fermentée ou non, à base de céréales, sont bien connues de l'homme du métier et peuvent varier selon la boisson fabriquée, en particulier selon que la boisson est une boisson fermentée ou non.

La prévention ou réduction du trouble de la boisson obtenue grâce à la présente invention étant obtenue du fait d'une activité enzymatique, l'étape d'ajout (i) d'au moins une cystéine endoprotéase telle que définie dans la section "*Cystéine endoprotéase*" ci-dessus ou (ii) d'un extrait de malt tel que défini dans la section "*Extrait de malt*" ci-dessus, peut être mise en oeuvre à n'importe quel stade du procédé de fabrication de la boisson qui n'est pas suivi d'une étape susceptible de détruire l'activité endoprotéasique.

De telles étapes susceptibles de détruire l'activité endoprotéasique sont bien connues de l'homme du métier, et incluent par exemple des étapes de traitement à des températures élevées, en particulier à des températures supérieures à 62°C, en particulier supérieures à 65°C, voire 68°C, telles que des étapes de saccharification ou d'ébullition.

Ainsi, dans un mode de réalisation particulier, l'étape d'ajout est mise en oeuvre après l'étape de saccharification et/ou d'ébullition du procédé de fabrication de la boisson.

Dans un mode de réalisation particulier, lorsque la boisson fabriquée est une boisson à base de céréales fermentée, en particulier lorsque la boisson fabriquée est une bière, ladite au moins une cystéine endoprotéase ou ledit extrait de malt est ajouté au cours de l'étape de fermentation du procédé de fabrication de la boisson, de préférence au début de l'étape de fermentation du procédé de fabrication de la boisson.

Comme il est bien connu de l'homme du métier, plusieurs types de fermentations peuvent être mises en oeuvre pour produire une boisson fermentée à base de céréales, en particulier une bière. Ainsi, ladite au moins une cystéine endoprotéase ou ledit extrait de malt peut être ajouté au cours d'une étape de fermentation basse, au cours d'une étape de fermentation haute ou au cours d'une étape de fermentation spontanée.

De préférence, la boisson en cours de fabrication est ainsi mise en contact avec ladite au moins une cystéine endoprotéase ou ledit extrait de malt pendant une durée appropriée pour permettre aux enzymes de dégrader les protéines responsables du trouble. Une telle durée dépendra des conditions d'incubation de la cystéine endoprotéase ou de l'extrait de malt avec la boisson en cours de fabrication, en particulier des conditions de température et de pH.

Les inventeurs ont montré que la durée habituelle de l'étape de fermentation au cours du procédé de fabrication d'une bière, qu'il s'agisse d'une fermentation basse ou haute, était particulièrement appropriée pour obtenir une prévention ou diminution du trouble de la bière telle que définie dans la section "*Diminution du trouble*" ci-dessus.

Ainsi, dans un mode de réalisation particulier, la boisson en cours de fabrication est mise en contact avec ladite au moins une cystéine endoprotéase ou ledit extrait de malt pendant 8 à 12 jours, de préférence pendant 10 jours, à une température comprise entre 10 et 12°C, de préférence à une température de 12°C, de préférence à un pH de départ de 5,3-5,4, le pH final pouvant atteindre 4,2-4,3 du fait de l'évolution naturelle du pH au cours de la fermentation.

Dans un autre mode de réalisation particulier, la boisson en cours de fabrication est mise en contact avec ladite au moins une cystéine endoprotéase ou ledit extrait de malt pendant 4 à 8 jours, de préférence pendant 6 jours, à une température comprise entre 20 et 25°C, de préférence à un pH de départ de 5,3-5,4.

Typiquement le procédé de fabrication de bière selon l'invention comprend les étapes suivantes :
a) une étape de maltage ou de fourniture du malt,
b) une étape de concassage ou broyage du malt obtenu à l'étape a),
c) une étape de mélange du malt broyé à l'étape b) avec de l'eau de préférence tempérée pour former une maische,
d) une étape de saccharification de la maische obtenue à l'étape c) pour former un moût, de préférence par décoction, infusion par palier ou infusion simple,
e) une étape de filtration pour obtenir le moût primitif,
f) une étape d'ébullition comprenant une étape de houblonnage amérisante et aromatisante,
g) une étape de séparation tangentielle "whirlpool" ou par centrifugation pour séparer le trouble du moût,
h) une étape de refroidissement, d'ensemencement et d'oxygénation du moût,
i) une étape de fermentation, en particulier basse, haute ou spontanée, en présence de levures, au cours de laquelle, de préférence au début de laquelle, ladite au moins une cystéine endoprotéase ou ledit extrait de malt est ajouté,
j) une étape de garde du produit fermenté obtenu à l'étape g),
k) une étape de filtration et séparation des levures, et
l) une étape de conditionnement.

Les étapes a) à h) et j) à I) peuvent être mises en oeuvre de manière conventionnelle dans des conditions bien connues de l'homme du métier.

Lors de l'étape i) de fermentation, le moût est de préférence mis en contact avec ladite au moins une cystéine endoprotéase ou ledit extrait de malt pendant 8 à 12 jours, de préférence pendant 10 jours, à une température comprise entre 8 et 14°C, de préférence à une température de 12°C, de préférence à un pH de départ de 5,3-5,4, ou pendant 4 à 8 jours, de préférence pendant 6 jours, à une température comprise entre 15 et 20°C, de préférence à un pH de départ de 5,3-5,4.

Les procédés selon l'invention peuvent en outre comprendre une étape d'ajout d'une enzyme auxiliaire permettant de réduire ou de prévenir la formation d'un trouble. De tels enzymes auxiliaires permettant de réduire ou de prévenir la formation d'un trouble sont bien connues de l'homme du métier, et incluent par exemple le Brewers Clarex® commercialisé par DSM, ou la papaïne.

Les boissons obtenues par les procédés selon l'invention diffèrent des boissons obtenues par les procédés de fabrication de l'état de la technique dans la mesure où elles contiennent la cystéine endoprotéase ou l'extrait de malt introduits au cours des procédés selon l'invention, que les enzymes soient inactivées ou pas, et ladite cystéine endoprotéase ou ledit extrait de malt modifient la composition en peptides et protéines du produit obtenu, permettant la prévention ou diminution de la formation du trouble, habituellement dû à la présence de protéines résiduelles.

La présente invention a donc également pour objet une boisson à base de céréales, en particulier fermentée ou non, telle que définie à la section "*Boisson à base de céréales*" ci-dessus, de préférence une bière, susceptible d'être obtenue par les procédés selon l'invention.

La présente invention sera illustrée plus en détail par les figures et exemples ci-dessous.

### Brève description des figures

**Figure 1** : Schéma récapitulatif du protocole d'obtention des extraits de malt d'orge mis en oeuvre dans l'exemple 3.

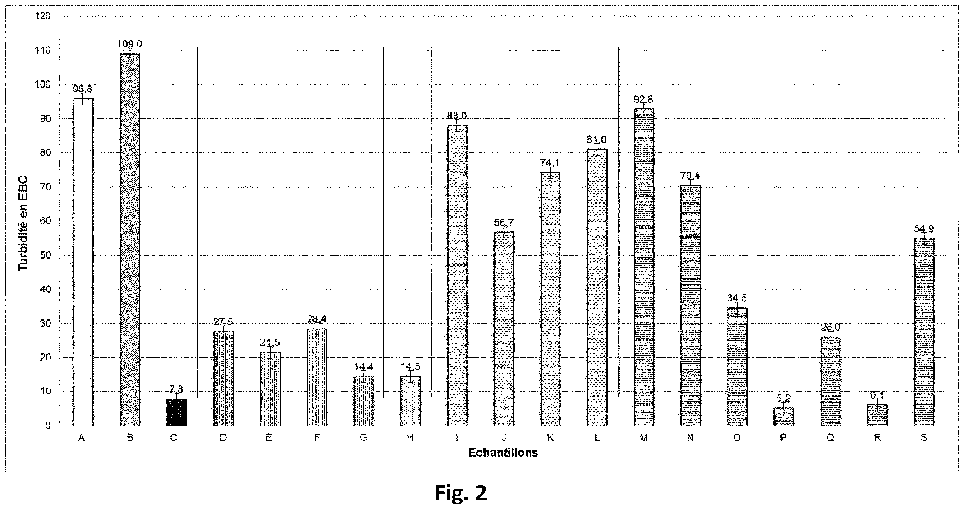

**Figure 2** : Effet sur le trouble colloïdal de la bière (test Chapon) des extraits et fractions obtenues dans l'exemple 3. A : Témoin négatif (1 mL de Tp Citrate 0,1 M pH 4,3 + 1 mL Tp Tris 50 mM pH 7,5); B : Témoin négatif (1 mL Tp Citrate 50 mM pH 5) ; C : Témoin positif (190 µL de Brewers Clarex® dilué 1/25 + 1 mL Tp Citrate 0,1 M pH 4,3) ; D : extrait brut (EB) ; E : surnageant 15% SA pH 4,3 ; F : C 65% SA dia (Tp Citrate 50 mM pH 5) ; G : non retenu (NR) sur colonne échangeuse de cations (SP) ; H : avant colonne DEAE (NR SP dia pH 7,5) ; I : NR DEAE 42-82 ; J : NR DEAE 83-95 ; K : NR DEAE 96-150 : L : NR DEAE 151-fin ; M : fraction 16 ; N : fraction 21 : O : fraction 25 ; P : fraction 31 ; Q ; fraction 38 ; R : fraction 46 ; S : fraction 55.
**Figure 3** : Chromatogramme de l'hydrolyse des gliadines avec DTT par les enzymes du malt d'orge et le Brewers Clarex® en HPLC sur Luna C18 décrite dans l'exemple 3.
**Figure 4** : Zoom d'un chromatogramme de l'hydrolyse des β-lactoglobulines avec DTT par les enzymes du malt d'orge et le Brewers Clarex® en HPLC sur Luna C18 décrite dans l'exemple 3.
**Figure 5** : Cinétique sur 6 h de l'hydrolyse du peptide Z-Gly-Pro-pNA par les enzymes du malt d'orge et le Brewers Clarex® à pH 4 décrite dans l'exemple 3.
**Figure 6** : Cinétique sur 6 h de l'hydrolyse du peptide Z-Gly-Pro-pNA par les enzymes du malt d'orge et le Brewers Clarex® à pH 5 décrite dans l'exemple 3.
**Figure 7** : Cinétique sur 6 h de l'hydrolyse du peptide Z-Phe-Arg-pNA par les enzymes du malt d'orge et le Brewers Clarex® à pH 4 décrite dans l'exemple 3.
**Figure 8** : Cinétique sur 6 h de l'hydrolyse du peptide Z-Phe-Arg-pNA par les enzymes du malt d'orge et le Brewers Clarex® à pH 5 décrite dans l'exemple 3.
**Figure 9** : Effet sur le trouble colloïdal de la bière (test Chapon) des différents extraits testés dans l'exemple 1.
**Figure 10** : Effet sur le trouble colloïdal de la bière (test Chapon) des différents extraits testés dans l'exemple 2.
**Figure 11** : Chromatogramme de l'hydrolyse des gliadines par les enzymes du malt d'orge et l'extrait brut de malt en HPLC sur Luna C18 décrite dans l'exemple 4. L'absorbance est mesurée à 214 nm.
**Figure 12** : Effet sur la fermentation de l'extrait de malt décrit dans l'exemple 5.

**Description des séquences**

| **SEQ ID NO**: | **Description** |
|---|---|
| 1 | Séquence protéique d'un premier variant de l'EP-A d'orge |
| 2 | Séquence protéique d'un deuxième variant de l'EP-A d'orge |
| 3 | Séquence protéique d'un premier variant de l'EP-B d'orge |
| 4 | Séquence protéique d'un deuxième variant de l'EP-B d'orge |
| 5 | Chaine mature d'un premier variant de l'EP-A d'orge |
| 6 | Chaine mature d'un deuxième variant de l'EP-A d'orge |
| 7 | Chaine mature d'un premier variant de l'EP-B d'orge |
| 8 | Chaine mature d'un deuxième variant de l'EP-B d'orge |

### Exemples

### Exemple 1 : Diminution du trouble de la bière avec différents types de malt d'orge

Cet exemple montre qu'un extrait de malt obtenu de différents types d'orge peut être utilisé pour clarifier la bière.

### Matériel et méthodes

### Extraction

50 g de malt fini de différents types d'orge ont été extraits dans 200 mL de Tampon citrate 0,1 M pH 4,3 conservé au réfrigérateur à 4°C. Ils ont été mixés dans un blender pendant 40 secondes à vitesse maximale, puis centrifugés à 3660 rpm pendant 10 min à 14°C. Le surnageant a été récupéré et filtré avec un papier filtre pour retirer les particules en suspensions.

### Bière non stabilisée

La bière utilisée dans cet exemple est la Bière Fink'bräu 33 cL en cannette conservée à température ambiante (21 °C), filtrée sur papier filtre, et dégazée pendant 20 min dans un bain à ultra-sons.

### Echantillons

Le Témoin positif (T+) correspond à 0,75 mL de Brewers Clarex® dilué au 1/25 + Bière non stabilisée qsp 50 mL.

Le Témoin négatif (T-) correspond à 4 mL de Tampon citrate 0,1 M pH 4,3 + qsp 50 mL de bière non stabilisée.

Les échantillons testés dont des extraits des malts d'orge suivants: Metaxa 3086 Ngt 1, Metaxa 3352 Ngt 1, Esterel 23036 B2PI, Esterel 23145 B1PO, Arturio 23005 B2PR, Arturio 23182 B1PO, Azurel 23054 B2PR, Azurel 23108 B2PR, Cervoise 23094 Ngt 1, Cervoise 3453 Ngt 1, Cartel 2965 Ngt 1, Cartel 3259 Ngt 1, Béatrix 23050 B2SA, Béatrix 23189 B2SA, Sébastian 993 Ngt 2, Sébastian 23119 B2PI, Grace 23076 B3ST, Grace 23219 B3ST, Prestige 23077 B3ST, Chill 23151 B3ST, Tipple 23192 B2PR, Tipple 23249 B2SA, Chamonix 404 Ngt 2, Chamonix 3227 Ngt 1, Charmey 436 Ngt 2, Charmey 897 Ngt 2, Henley 3319 Ngt 1, Henley 3328 Ngt 1.

Les échantillons testés sont constitués de 4 mL d'extrait + Bière non stabilisée qsp 50 mL.

Les mélanges ont été incubés pendant 17h au bain-marie à 37°C.

### Test Chapon

Les Témoins et les échantillons ont été incubés à 37°C pendant au minimum 5h. Dans des tubes à essai en plastique transparents (polystyrène), 0,6 mL d'éthanol 96% (=6%) ont été mélangés avec qsp 10 mL d'échantillon (x2).

Le mélange a été incubé 30 min à -8°C dans un bain cryostaté.

La turbidité a été mesurée au turbidimètre à -8°C.

### Résultats

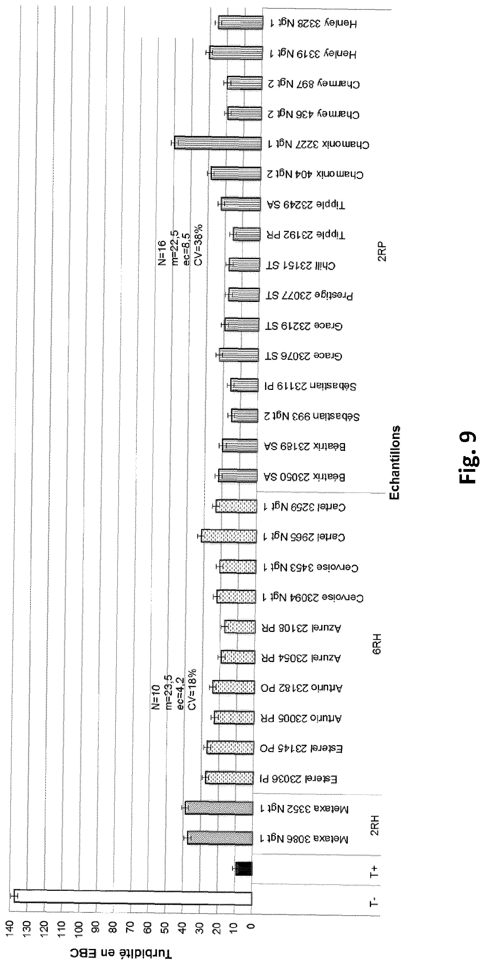

Les résultats sont présentés dans la **Figure 9**. Ces résultats montrent qu'une diminution de la turbidité est observée quel que soit le type d'orge à partir duquel l'extrait de malt a été obtenu.

### Exemple 2 : Diminution du trouble de la bière avec du malt de blé

Cet exemple montre qu'un extrait de malt de blé peut être utilisé pour clarifier la bière.

### Matériel et méthodes

### Extraction

50 g de malt fini de différents types d'orge ont été extraits dans 200 mL de Tampon citrate 0,1 M pH 4,3 conservé au réfrigérateur à 4°C. Ils ont été mixés dans un blender pendant 40 secondes à vitesse maximale, puis centrifugés à 3660 rpm pendant 10 min à 14°C. Le surnageant a été récupéré et filtré avec un papier filtre pour retirer les particules en suspensions.

### Bière non stabilisée

La bière utilisée dans cet exemple est la Bière Fink'bräu 33 cL en cannette conservée à température ambiante (21 °C), filtrée sur papier filtre, et dégazée pendant 20 min dans un bain à ultra-sons.

### Echantillons

Le Témoin positif (T+) correspond à 0,75 mL de Brewers Clarex® dilué au 1/25 + Bière non stabilisée qsp 50 mL.

Le Témoin négatif (T-) correspond à 4 mL de Tampon citrate 0,1 M pH 4,3 + qsp 50 mL de bière non stabilisée.

Les échantillons testés dont des extraits de malt d'orge et de blé suivants: Malt d'orge Chill 22304 B2BR, Malt d'orge Arturio 23175 B1CA, Malt d'orge diastasique Arturio 23112 B2BR, Malt de Blé Apache 23086 (Arcis sur Aube) et Malt de Blé Bagou (Arcis sur Aube).

Les échantillons testés sont constitués de 0,5, 1, 2, 3 ou 4 mL d'extrait + Bière non stabilisée qsp 50 mL.

Les mélanges ont été incubés pendant 17h au bain-marie à 37°C.

### Test Chapon

Les Témoins et les échantillons ont été incubés à 37°C pendant au minimum 5h. Dans des tubes à essai en plastique transparents (polystyrène), 0,6 mL d'éthanol 96% (=6%) ont été mélangés avec qsp 10 mL d'échantillon (x2).

Le mélange a été incubé 30 min à -8°C dans un bain cryostaté.

La turbidité a été mesurée au turbidimètre à -8°C.

### Résultats

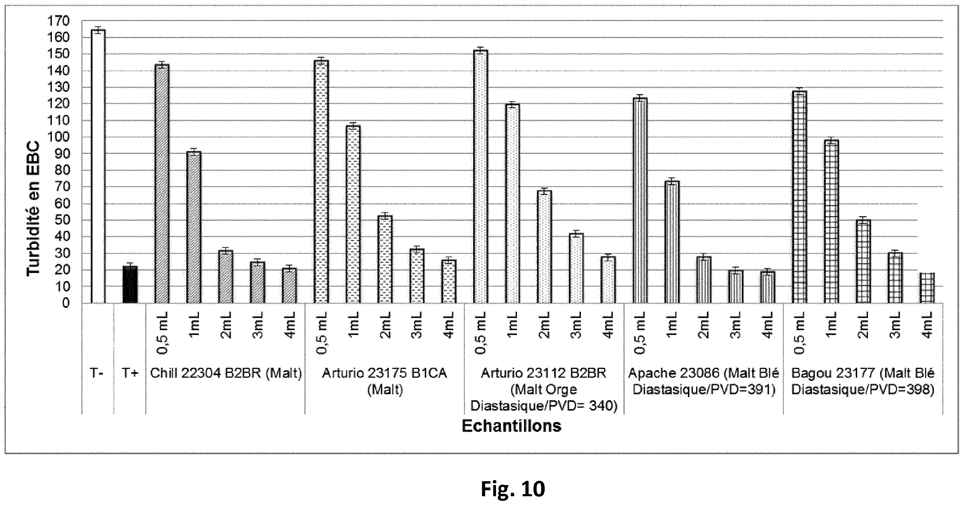

Les résultats sont présentés dans la **Figure 10**. Ces résultats montrent qu'une diminution de la turbidité est également observée avec du malt de blé diastasique, ainsi que du malt d'orge diastasique.

### Exemple 3 : Identification dans l'extrait de malt d'enzymes responsables de l'effet sur la turbidité

Cet exemple montre l'effet d'extraits de malt sur le trouble de la bière et l'implication de cystéine endoprotéases d'orge dans cet effet.

### Matériels et méthodes

### Matériels

Le malt utilisé pour les différentes expériences provient de grains d'orge de la variété Beatrix *(Hordeum vulgare)* maltés par les malteries Soufflet (Nogent sur Seine /Aube) mais les enzymes sont présentes dans toutes les variétés d'orge qu'elles soient d'hiver (deux ou six rangs) ou de printemps.

Le malt a été broyé dans un vibrating feeder L 24 de chez Fritsch (Allemagne).

Les différents produits utilisés pour les tampons et les gels d'électrophorèses proviennent des fournisseurs Alfa Aesar, Carbo Erba et Sigma. L'acrylamide 40% vient de chez Fisher Scientific.

Le dosage colorimétrique des protéines a été réalisé avec le kit BC Assay d'Uptima Interchim.

Les chromatographies échangeuses d'ions ont été faites sur des FPLC Akta explorer 100 et Akta Prime (GE Healthcare, USA) avec des colonnes XK 26/10 et des gels Sepharose SP Fast Flow (FF) 50 ml et Sepharose DEAE FF (GE Healthcare, USA). La séparation est également faisable sur d'autres types de colonnes échangeuses de cations et d'anions.

Les gels filtrations ont été faites sur des FPLC Akta explorer 10 (Amersham Pharmacia Biotech) et Akta explorer 100 (GE Healthcare, USA) avec des colonnes Sephadex HR (High Resolution) S200 (16/60) et Superose 12 (10/30).

La détection des protéines a été effectuée par spectroscopie à 3 longueurs d'onde : 215, 260 et 280 nm.

Les analyses en HPLC ont été réalisées sur un appareil Alliance HT Waters 2795 separation module/ Waters 2487 Dual λ absorbance detector, et la colonne utilisée était une Luna 5µ (C18) 100Å (250 x 4,60 mm) (Phenomenex). La détection des protéines a été effectuée par spectroscopie à 2 longueurs d'onde : 214 et 280 nm.

Les échantillons ont été analysés par spectrométrie de masse, après hydrolyse trypsique, sur nano LC-MS/MS ESI ORBITRAP Velos. Les analyses peptidiques ont été réalisées avec le logiciel Mascot 2.2.

La modélisation des enzymes a été réalisée avec le logiciel Modeller 9.14.

La centrifugeuse est une Beckman Avanti (USA) J26 XP (rotor : FiberLite® F10BCI - 6x500y)

Le trouble a été mesuré sur un tannometer Pfeuffer (Allemagne) après passage dans un cryothermostat à immersion Hubert Variostat CC. La bière témoin non stabilisée est une Finkbräu (Lidl) et le témoin positif est le Brewers Clarex® (DSM, Pays-Bas).

Les gliadines (totales) ainsi que les β-lactoglobulines utilisées pour les tests d'hydrolyse ont été fournies et purifiées par l'INRA de Nantes (Unité BIA centre Angers-Nantes / Loire Atlantique).

Le peptide Z-GP-pNA ainsi que le peptide Z-FR-pNA proviennent de chez Bachem (Suisse).

La lecture de la DO a été faite par un lecteur de plaques Epoch (Biotek, USA).

Les membranes de dialyse étaient des membranes visking (Medicell Int.) de seuil de coupure 12-14000 Da.

### Préparation des tampons

Le tampon citrate 0,1 M pH 4,3 a été préparé avec de l'acide citrique 0,1 M (PM = 210,14 g/mol L⁻¹) et ajusté à pH 4,3 par l'ajout de citrate de sodium à 0,1 M (PM = 294,1 g/mol L⁻¹). Pour les autres tampons citrate utilisés, ils ont été préparés également avec les solutions à la molarité indiquée et ajustés au pH voulu par ajout de citrate de sodium.

Le tampon acétate 50 mM pH 4 a été préparé avec de l'acide acétique 50 mM et ajusté à pH 4 par l'ajout d'acétate de sodium à 50 mM (PM = 136,08 g/mol L⁻¹).

Le tampon Tris/HCl 50 mM pH 7,5 a été préparé avec du Tris 50 mM et ajusté à pH 7,5 par l'ajout d'acide chlorhydrique (HCl).

Le tampon acide citrique 0,1 M/ disodium phosphate 0,2 M (PM = 177,99 g/mol L^{- 1}) a été préparé avec de l'acide citrique 0,1 M et ajusté à pH 4 (ou 5) par l'ajout de disodium phosphate 0,2 M.

Le tampon pour le test de lyophilisation a été préparé avec du tampon Tris/HCl 100 mM pH 8,5 (PM T5ris = 121,14 g/mol L⁻¹) dans lequel a été ajouté de l'EDTA (Ethylene diamine tetra acetic acid) 5 mM (PM = 292,24 g/mol L⁻¹), de la cystéine 2 mM (PM = 121,2 g/mol L⁻¹), 4% de mannitol (p/v) et 1% de saccharose (p/v)

### Préparation de l'extrait

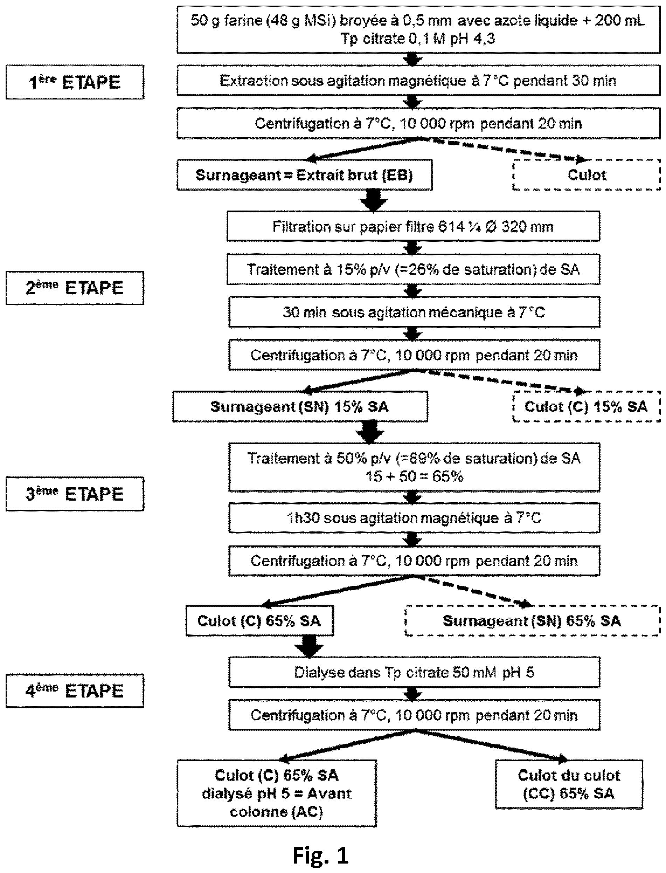

Les grains de malt ont été broyés sous azote liquide à 0,5 mm. 48 g de MSi (Matière Sèche initiale) de farine ont été mis en suspension dans 200 mL de tampon citrate 0,1 M pH 4,3 sous agitation magnétique à 7°C pendant 30 minutes. Après centrifugation à 10 000 rpm, pendant 20 minutes à 7°C, le surnageant a été récupéré et filtré sur papier filtre. Il est dénommé « extrait brut » (EB). Cet EB a été traité à 26% de saturation au sulfate d'ammonium ((NH₄)₂ SO₄) (SA) soit 15% p/v sous agitation magnétique à 7°C pendant 30 minutes. S'en est suivie une seconde centrifugation à 10 000 rpm, pendant 20 minutes à 7°C. Le surnageant a été récupéré et filtré sur papier filtre. Ainsi le « surnageant traité à 15% de sulfate d'ammonium » (SN-15%-SA) a été obtenu. Du SA a été rajouté au SN-15%-SA de façon à atteindre 89% de saturation soit 65% p/v final. Après 1H30 à 7°C sous agitation magnétique à 7°C l'extrait a été centrifugé de nouveau à 10 000 rpm, pendant 20 minutes à 7°C. Le surnageant a été éliminé et le culot obtenu a été remis en suspension dans 50 mL de tampon citrate 0,1 M pH 4,3 et dialysé tout d'abord 30 minutes dans 2 L d'eau distillée, suivie de deux bains de 2 h chacun dans 2 L de tampon citrate 50 mM pH 5 puis une nuit contre 5 L de tampon citrate 50 mM pH 5 afin d'obtenir l'extrait final appelé C65%SA dia pH 5 (Culot 65% Sulfate d'ammonium dialysé pH 5) qui est également l'« Avant Colonne » (AC) pour le passage sur la SP Sepharose FF (**Figure 1**).

### Test Chapon

L'ensemble des tests d'efficacité de réduction de la turbidité de la bière a été fait via le test Chapon (Chapon (1993) J. Inst. Brew. 99:49-56). 2 mL d'échantillon ont été mélangés à 25 mL qsp de bière non stabilisée puis incubés à 37°C pendant au minimum 5 h dans un bain Marie. Dans des tubes à essai en polystyrène, 9,4 mL d'échantillon incubés ont été prélevés et 0,6 mL d'éthanol 96% ont été ajoutés. Les essais ont été faits en duplicata. Après incubation pendant 30 min à -8°C dans un bain cryostaté la turbidité a été mesurée à -8°C sur un tannometer Pfeuffer. Les résultats sont exprimés en EBC (European Brewery Convention) (0,25 EBC = 1 NTU (Nephelos Turbidity Unit)).

### Chromatographies échangeuses d'ions

*Chromatographie échangeuse de cations* - Dans un premier temps, l'extrait (C65%SA dialysées à pH 5) a été déposé sur une colonne (XK 26/10) Sepharose SP FF équilibrée dans du tampon citrate 50 mM pH 5 (= tp A), débit= 13 ml/min (147 cm/h). L'élution a été effectuée en augmentant la force ionique par un gradient linéaire de 0 à 50% de tampon B (tp A + NaCI 1 M) en 25 CV (Volume de Colonne). Après un plateau à 50% de B pendant 2 CV, la colonne a été rincée à 100% B en 7 CV, puis rééquilibrée en tampon A. Des fractions de 15 ml ont été collectées. Seule la fraction « non retenue » active au test Chapon a été récupérée (NR SP).

*Chromatographie échangeuse d'anions* - Après dialyse deux fois 2 h puis 1 nuit, dans des bains de 5 L de tampon Tris 50 mM pH 7,5 (= tp A2), le NR SP a été déposé sur une colonne Sépharose DEAE FF, équilibrée par le tampon A2, débit = 5 ml/min et l'élution a été effectuée par un gradient linéaire de 0 à 30% de tampon B2 (Tris 50 mM pH 7,5 + NaCI 1 M) en 80 min puis par un palier de 10 min à 30% et un palier de 20 min à 100% de tampon B2. Les fractions collectées étaient de 2 mL.

### Gel filtration

*Colonne Sephadex S200* - 5 mL (soit 4,4 mg de protéines) de la fraction SP/DEAE contenant l'EP-B ont été déposés sur la colonne Sephadex S200. Le débit était à 0,5 mL/min et le tampon utilisé était du tampon citrate 50 mM pH 4,5. Les fractions collectées étaient de 5 mL.

*Colonne Superose 12* - 1,5 mL (soit 1,75 mg de protéines) de la fraction SP/DEAE contenant l'EP-A ont été déposés sur la colonne Sephadex S200. La fraction a été préalablement concentrée sur cellule Amicon Ultra 10KDa de 0,5mL (Millipore, Irlande). Le débit était de 0,8 mL/min et le tampon utilisé était du tampon citrate 50 mM pH 4,5. Les fractions collectées étaient de 2 mL.

### Electrophorèses (SDS-PAGE, PAGE, zymogrammes, tricine)

*SDS-PAGE-* Les différents échantillons ont été analysés en SDS-PAGE avec un gel de séparation à 12% d'acrylamide et un gel de concentration à 5% selon la technique de Laemmli (Laemmli (1970) Nature 227:680-685*)*. La migration a été réalisée à température ambiante dans une cuve à électrophorèse verticale Atto sous 10 mA dans le gel de concentration puis 20 mA dans le gel de séparation. Selon les gels, les colorations ont été faites au bleu de Coomassie R250 ou au bleu de Coomassie G250. Dans les puits, 5 µL de standards de poids moléculaires connus et 20 µL d'échantillon ont été déposés. Avant dépôt, les échantillons ont été préparés comme suit : 40 µL d'échantillon + 20 µL de solution de dénaturation (Tris 50 mM pH 6,8, glycérol 20%, SDS 4%, bleu de bromophénol 0,01%) + 3 µL de β-mercapto-éthanol.

*Native PAGE-* Le protocole était le même que pour les gels PAGE excepté la présence du SDS.

*SDS-Tricine PAGE-* Les SDS-tricine PAGE ont été réalisés selon le protocole de Schägger et von Jagow (1987) Anal. Biochem. 166:368-379, sur des gels à 10% d'acrylamide pour la séparation avant analyse des bandes par spectrométrie de masse ou à 16% pour l'étude de l'hydrolyse des hordéines. Les gels ont été colorés au bleu de Coomassie G250.

*Zymogrammes* - Les zymogrammes ont été réalisés sur des gels PAGE 12% dans lesquels ont été incorporées 0,1% de gliadines selon le protocole de Prabucka et Bielawski (2004) Acta Physiol. Plant. 26:383-391. Avant coloration au bleu de Coomassie R 250, le gel a été rincé 2 fois 1 h dans du tampon citrate 0,1 M pH 4,3 puis incubé dans du tampon citrate 0,1 M pH 4,3/ 4 mM de cystéine à 40°C durant une nuit.

### Analyse en spectrométrie de masse

Après SDS-Tricine-PAGE et coloration au bleu de Coomassie G250, les bandes d'intérêt ont été découpées au cutter en conditions stériles, puis analysées par la plateforme BIBS de l'INRA de Nantes (http://www.bibs.inra.fr).

### Hydrolyse des hordéines, gliadines et β-lactoglobulines

*Hydrolyse des hordéines : extraction* - 25 g de farine de grains d'orge broyés à 0,5 mm ont été mélangés sous agitation magnétique avec 200 mL de NaCI 0,5 M pendant 1h à température ambiante. Le mélange a ensuite été centrifugé à 10 000 rpm pendant 30 minutes à 12°C. Le surnageant a été éliminé et le culot a été suspendu dans 200 mL de NaCI 0,5 M et mis sous agitation magnétique pendant 1 h à température ambiante. Après avoir été centrifugé à 10 000 rpm pendant 30 minutes à 12°C, le surnageant a été éliminé et le culot a été congelé à -20°C puis lyophilisé.

Le lyophilisat a été remis en suspension dans du propanol à 50% (15 mL/g) à 60°C sous agitation magnétique pendant 45 min puis centrifugé à 10 000 rpm pendant 30 minutes à 12°C. Le surnageant appelé « surnageant propanol » a été conservé à 4°C et le culot a de nouveau été extrait avec du propanol à 50%.

Le culot a ensuite été mis en suspension dans du propanol 50% (15 mL/g) + 2% de β-mercapto-éthanol (βME) à 60°C sous agitation magnétique pendant 45 minutes. Il a ensuite été centrifugé à 10 000 rpm pendant 30 minutes à 12°C. Le surnageant dénommé « surnageant propanol/ β-mercapto-éthanol » a été conservé à 4°C. Le culot a subi cette étape deux fois de plus.

Les surnageants propanol et propanol/ βME ont été dialysés séparément contre 20 L d'eau distillée (3 bains de 2 h et une nuit) puis centrifugés à 10 000 rpm pendant 30 minutes à 12°C.

Les culots ainsi que les surnageants ont été congelés à -20°C puis lyophilisés. Les lyophilisats ont ensuite été broyés en une fine poudre et stockés à -20°C.

A la fin de l'extraction, les Hordéines Propanol (HP) et les Hordéines Propanol/ β-mercapto-éthanol (HPβME) ont été obtenues (Koehler et Ho (1990) Plant Physiol. 94251-258; Wrobel (1992) J. Inst. Brew. 98:471-478; Zhang et Jones (1996) Planta 199:565-572).

Ces extraits étant difficilement solubles, ils ont été délipidés avant analyse. Les HP ont été délipidées selon le protocole suivant : dans un tube en verre de 15 mL, 0,302 g de HP ont été pesés et mises en suspension dans 10 mL de Dichlorométhane + 5 mL d'acétone. Après mélange au vortex pendant 20 minutes et centrifugation à 4°C pendant 10 minutes à 3000 rpm, le surnageant a été éliminé et le culot a été traité une seconde fois puis séché sous vide et conservé à -20°C. L'extrait obtenu est appelé HPdl.

*Hydrolyse des hordéines: analyse en SDS-PAGE et HPLC* - Après avoir mis en suspension les hordéines (HPdl) (1 mg/mL) dans du tampon sodium-succinate 20 mM pH 4,5 contenant 10 mM de β-mercapto-éthanol, 2 µg de protéines ont été ajoutées pour la fraction contenant l'EP-B ainsi que pour le témoin positif (Brewer Clarex®). L'incubation a été faite à 40°C. 20 µL du mélange réactionnel ont été prélevés à différents temps (T= 0/5/15/30/60/90min/2h/4h et 24h), dilués dans le tampon de dénaturation, portés à ébullition pour stopper la réaction, puis congelés à -20°C avant analyse sur un gel SDS-Tricine-PAGE 16%. Dans le cas de l'analyse par HPLC en phase réverse 100µl de l'extrait 24 h ont été prélevés.

### Hydrolyse des gliadines et de la β-lactoglobuline : analyse par HPLC en phase réverse -

Dans le cas des gliadines et de la β-lactoglobuline, une solution à 1 mg/mL a été préparée dans du tampon acétate 50 mM pH 4. Des essais ont aussi été effectués en ajoutant du DL-Dithiothreitol (DTT) à une concentration finale de 2 mM avant l'incubation. Pour initier la réaction d'hydrolyse, 2 et 5 µg de protéines (EP-B / EP-A et BC) ont été ajoutés dans 200 µL de substrat +/- 4 µL de DTT à 0,1 M. Le mélange réactionnel a été incubé à 40°C pendant 20 h.

### HPLC (High Performance Liquid Chromatography) en phase réverse

La colonne Luna C18 (Phenomenex) a été équilibrée par l'éluant A (H₂O milliQ + acide tri-fluoroacétique (TFA) 0,11%) et l'élution a été effectuée par un gradient linéaire de 0 à 50% d'éluant B (acétonitrile + TFA 0,09%) en 54 min à un débit de 1 mL/min pour l'hydrolyse des gliadines et des β-lactoglobulines et un gradient linéaire de 0 à 60% de B en 32 min à un débit de 0,8 mL/min pour l'hydrolyse des hordéines.

50 µL d'échantillon ont été injectés. La lecture a été faite à 214 nm et 280 nm et la température de la colonne a été maintenue à 50°C (Marchylo et Kruger (1984) Cereal Chem. 61:295-301; Marchylo et al. (1986) Cereal Chem. 63:219-231).

### Hydrolyse des peptides Z-Gly-Pro-pNA et Z-Phe-Arg-pNA

Pour chaque peptide testé, l'hydrolyse a été effectuée à pH 4 et pH 5 (Davy et al. (1998) Plant Physiol. 117:255-261; Simpson et al. (2001) Plant Sci. 161:825-838).

*Hydrolyse du peptide Z-Gly-Pro-pNA (Z-GP-pNA)* - 17,6 µg de protéines ont été mises à réagir pour chaque enzyme, soit EP-A, EP-B et BC avec qsp 500 µL de tampon acide citrique 0,1 M/ disodium phosphate 0,2 M (pH 4 ou 5) et 125 µL de substrat 10 mM (solubilisé dans le même tampon + 40% de dioxane). La concentration finale pour le Volume Réactionnel (VR) en substrat était donc de 2 mM et de 8% de dioxane.

Pour chaque échantillon, l'hydrolyse a été faite en duplicata (dépôt de 250 µL du VR dans une micro-plaque de 96 puits).

La libération du pNA a été suivie par lecture de la DO à 410 nm à différents temps (T= 0/ 10/ 20/ 30/ 40/ 50/ 60min et 1h30/ 2h/ 2h30/ 3h/ 3h30/ 4h/ 4h30/ 5h/ 5h30/ 6h et 24h).

*Hydrolyse du peptide Z-Phe-Arg-pNA (Z-FR-pNA)* - 17,6 µg de protéines ont été mis à réagir pour chaque enzyme, soit EP-A, EP-B et BC avec qsp 500 µL de tampon acétate 50 mM (pH 4 ou 5) + 5% DMSO et 125 µL de substrat à 10 mM solubilisé dans le même tampon. La concentration finale pour le Volume Réactionnel (VR) en substrat était donc de 2 mM.

Pour chaque échantillon, l'hydrolyse a été faite en duplicata (dépôt de 250 µL du VR dans une micro-plaque de 96 puits).

La libération du pNA a été suivie par lecture de la DO à 410 nm à différents temps (T= 0/ 10/ 20/ 30/ 40/ 50/ 60min et 1h30/ 2h/ 2h30/ 3h/ 3h30/ 4h/ 4h30/ 5h/ 5h30/ 6h et 24h).

### Test de lyophilisation

Après passage sur colonnes SP puis DEAE de l'extrait de malt, les fractions correspondant au pic de séparation de l'enzyme EP-B ont été réunies. Le volume final obtenu a été divisé en deux puis dialysé séparément pendant 30 minutes dans de l'eau distillée suivi de deux bains de 2 h puis d'une nuit à 4°C dans du tampon citrate 50 mM pH 4,2 pour l'un et dans du tampon Tris/ HCl/ EDTA/ Cystéine/ Mannitol/ Saccharose pour l'autre.

Le volume après dialyse a de nouveau été divisé en deux. La première portion a été conservée à -20°C en attente d'analyse au test Chapon et la seconde a été lyophilisée. Le lyophilisat a été mis en suspension dans un volume d'eau distillée égal au volume initial mis au lyophilisateur et a été analysé au test Chapon.

### Résultats

### Comparaison en Test Chapon de l'efficacité des extraits obtenus à chaque étape de l'extraction des enzymes du malt d'orge

Le Test Chapon permet en mesurant la diminution du trouble de sélectionner les fractions actives au cours de la purification. Ainsi après fractionnement au sulfate d'ammonium, les inventeurs ont montré que l'activité était conservée dans le SN15%SA dialysé puis dans le C65%SA dialysé (**Figure 2**). Le C15%SA dialysé et le SN65%SA dialysé ne présentent aucune activité. Un test Chapon effectué en ajoutant des quantités équivalentes de protéines (1 mg) montre une forte augmentation de l'activité dans le C65%SA dia (108 EBC pour l'EB; 50,2 EBC pour le C65%SA dia). Ainsi, le traitement au sulfate d'ammonium permet une première concentration et purification des enzymes d'intérêt.

### Séparation des enzymes du malt d'orge par chromatographies échangeuses d'ions

L'analyse par test Chapon des fractions éluées après chromatographie de l'extrait de malt (C65%SA dialysé pH 5) sur une colonne échangeuse de cations (SP) a montré que l'activité enzymatique se trouvait dans la fraction non retenue sur la colonne. Cette fraction a été ensuite séparée sur une colonne échangeuse d'anions (DEAE). Cette deuxième chromatographie a permis d'isoler deux fractions enzymatiques capables de diminuer la turbidité de la bière à 5,2 et 6,1 EBC (**Figure 2** : fractions 31 et 46).

### Analyse par électrophorèses (SDS-PAGE, PAGE, zymogrammes)

L'analyse par électrophorèse a montré que la fraction 31 était composée de plusieurs bandes protéiques dont un groupe principal aux environs de 35 kDa, une bande aux environs de 60 kDa et une bande plus fine aux environs de 30 kDa. La fraction 46 contenait 2 bandes de PM aux environs de 60 et 35 kDa. Le zymogramme a permis de confirmer la présence dans les fractions 31 et 46/47, d'enzymes capables d'hydrolyser des protéines riches en prolines (gliadines). Les enzymes dans ces 2 fractions avaient des mobilités différentes ce qui indique qu'elles étaient différentes.

### Séparation des enzymes du malt d'orge par gel filtration

Après séparation des enzymes d'intérêt du malt d'orge par chromatographies échangeuses d'ions, les inventeurs ont utilisé la chromatographie d'exclusion diffusion (gel filtration) afin d'affiner la pureté des fractions présentant l'activité enzymatique. La séparation de la fraction 31 sur la colonne Sephadex S200 a montré un pic principal précédé de deux épaulements. L'analyse en test Chapon et par zymogramme a révélé que l'enzyme était présente dans la fraction 1C4 correspondant au pic principal. Par électrophorèse SDS, les inventeurs ont constaté que cette fraction était constituée de plusieurs bandes protéiques aux environs de 35 kDa.

Le chromatogramme de la fraction 47 a montré plusieurs pics mal séparés. L'analyse en test Chapon et par zymogramme a révélé que l'enzyme était présente dans la fraction 5A5 correspondant au premier pic.

### Caractérisation des enzymes par spectrométrie de masse

Après SDS-PAGE les bandes protéiques des fractions 31 et 46 ont été découpées et analysées par spectrométrie de masse après hydrolyse trypsique. Dans le cas de la fraction 31, les 2 bandes de PM 30 kDa et 28 kDa correspondent à la forme mature d'une cystéine protéase : EP-B (Référence Uniprot : P25249 (SEQ ID NO: 3) ou P25250 (SEQ ID NO: 4)). Deux variants de cette protéase ont été décrits mais les différences entre ces 2 variants sont trop faibles pour que l'analyse permette de dire quel variant est présent. Les EP-B P25249 et P25250 sont des protéases de PM 25180 et 25318 et de pl 4,77 et 4,96. La fraction 31 contient également des β-amylases (bande de PM 60 000) et des serpines (groupe de bandes aux environs de 35 000).

Dans le cas de la fraction 46, la cystéine protéase a été retrouvée également dans 2 bandes de PM 30 et 28 kDa qui correspondent à la forme mature de la cystéine protéase EP-A (Référence Uniprot : O04675 (SEQ ID NO: 1) ou O04677 (SEQID NO: 2)). Comme pour l'EP-B, deux variants existent mais l'analyse ne permet pas de dire quel variant est présent. Sur la base des séquences des protéines matures, ces protéases ont des PM respectifs de 25590 et 25648 et des pl de 4,4 et 4,37. Les autres bandes observées sur l'électrophorégramme correspondent aussi à une β-amylase et des serpines.

Un alignement de séquences a montré que les séquences des deux EP-B et celles des deux EP-A présentent respectivement 98% et 99% d'acides aminés identiques. En revanche, les EP-A possèdent 52% d'homologie séquentielle avec les EP-B. Ces deux cystéines protéinases ont une structure semblable et possèdent un site catalytique identique composé de trois acides aminés : cystéine (28), aspartate (162) et histidine (163). Bien qu'ayant un effet similaire sur la diminution du trouble de la bière par rapport à l'enzyme témoin positif (Brewers Clarex®), elles n'en sont pas moins différentes. En effet, elles n'ont que 9 et 11% (respectivement) d'acides aminés identiques avec le Brewers Clarex®. Par ailleurs le nombre important de gap nécessaire à cet alignement montre que ces enzymes ne présentent aucune parenté structurale. Cette absence d'homologie structurale peut également être mise en évidence avec la modélisation 3D de l'enzyme.

### Hydrolyse des hordéines, gliadines et β-lactoglobulines

*Analyse en électrophorèse de l'hydrolyse des hordéines* - Le suivi de l'hydrolyse des hordéines par l'analyse en gel SDS-Tricine-PAGE n'a pas permis pas de conclure s'il existait une différence réelle de mécanisme d'action entre l'EP-B et le témoin positif, même si l'EP-B paraît plus efficace. En effet, il n'y a plus de bandes visibles après 24h de réaction alors que des bandes peuvent encore être observées après 24h en présence de Brewers Clarex.

*Analyse en HPLC de l'hydrolyse des hordéines* - La fraction enrichie en EP-B (fraction 31) est capable d'hydrolyser les hordéines et les dégrade différemment du témoin positif (Brewers Clarex®). En effet, les chromatogrammes ont montré que certains peptides générés étaient différents.

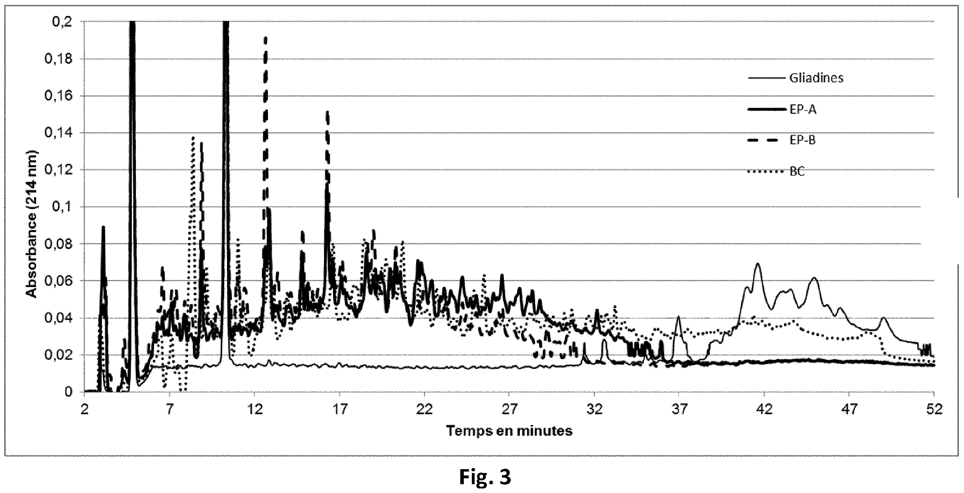

*Analyse en HPLC de l'hydrolyse des gliadines* - Dans le cas des gliadines, les chromatogrammes ont mis en évidence une action différente entre les enzymes du malt d'orge (EP-B et EP-A) et le témoin positif (Brewers Clarex®). On constate que l'hydrolyse (en présence de DTT) par les cystéines protéinases est totale, contrairement à celle avec le Brewers Clarex (BC). De très nombreux peptides hydrophiles ont été générés par l'hydrolyse par les différentes enzymes rendant difficile la comparaison des chromatogrammes. On peut tout de même voir quelques différences aussi bien entre le Brewers Clarex et les deux protéases du malt d'orge qu'entre les deux protéases (**Figure 3**).

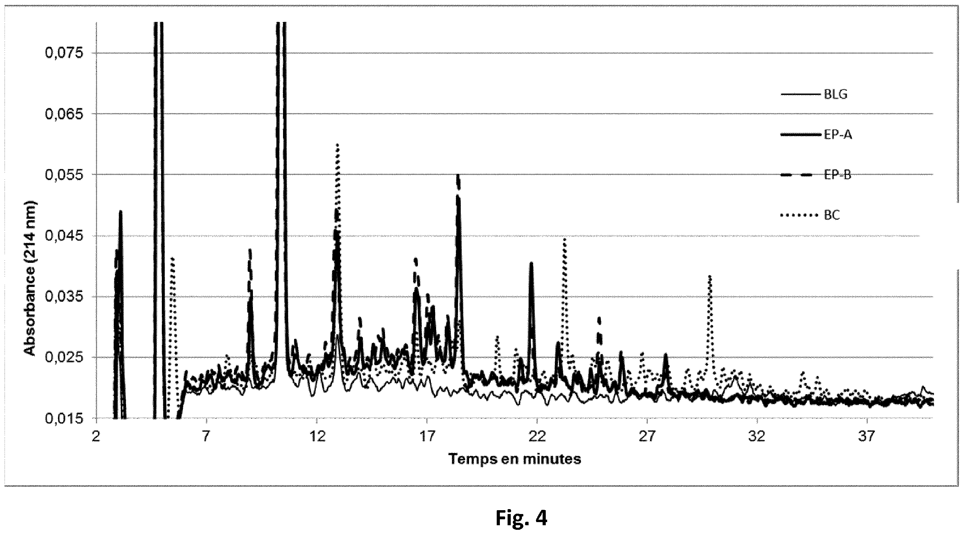

*Analyse en HPLC de l'hydrolyse des β-lactoglobulines* - Dans le cas de l'hydrolyse des β-lactoglobulines, les chromatogrammes ont également montré une action différente entre les enzymes du malt d'orge (EP-B et EP-A) et le témoin positif (Brewers Clarex®). Cette différence est visible avec ou sans DTT (**Figure 4**).

### Hydrolyse des peptides Z-Gly-Pro-pNA et Z-Phe-Arg-pNA

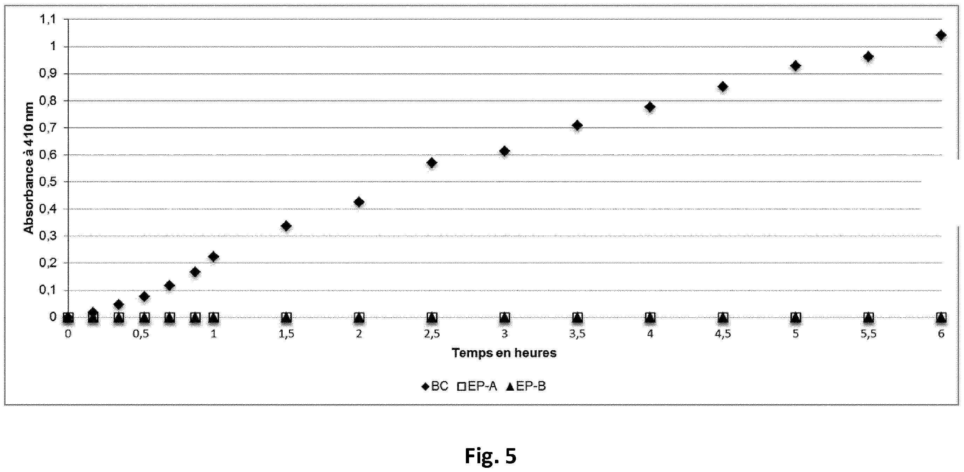

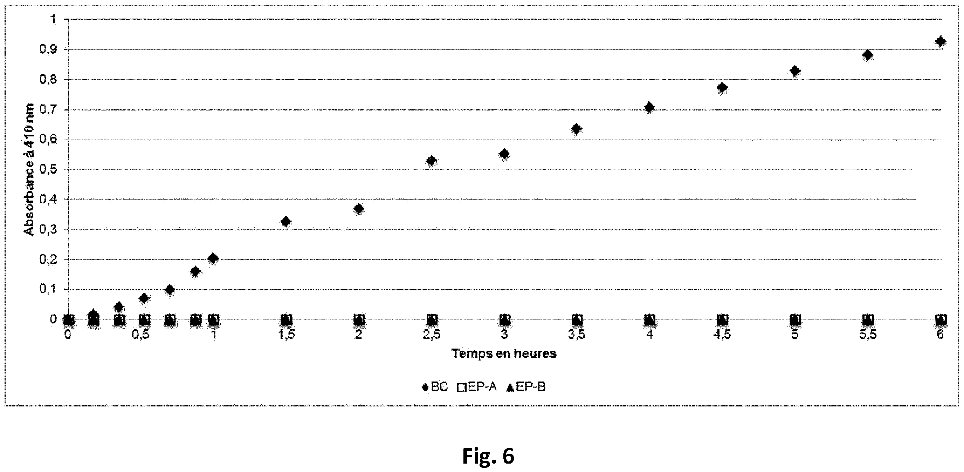

*Hydrolyse du peptide Z-Gly-Pro-pNA* - Seul le Brewers Clarex® a été capable d'hydrolyser le peptide Z-GP-pNA dans les tests réalisés (**Figures 5** **et** **6**). De plus, le Brewers Clarex est tout aussi efficace à pH 4 qu'à pH 5. Pour les enzymes du malt d'orge : EP-A et EP-B les DO sont nulles et ce même après 24h d'incubation, que la réaction soit faite à pH 4 ou 5.

Cette expérience met en évidence que le site d'action du Brewers Clarex est différent de celui des cystéines protéases EP-A et EP-B.

*Hydrolyse du peptide Z-Phe-Arg-pNA* - Dans le cas du peptide Z-FR-pNA, l'hydrolyse est uniquement visible avec l'enzyme EP-B. Il n'y a pas de dégradation avec l'EP-A ou le Brewers Clarex. De plus, l'EP-B est plus efficace à pH 4. En effet, la DO à pH 5 est deux fois moins élevée qu'à pH 4.

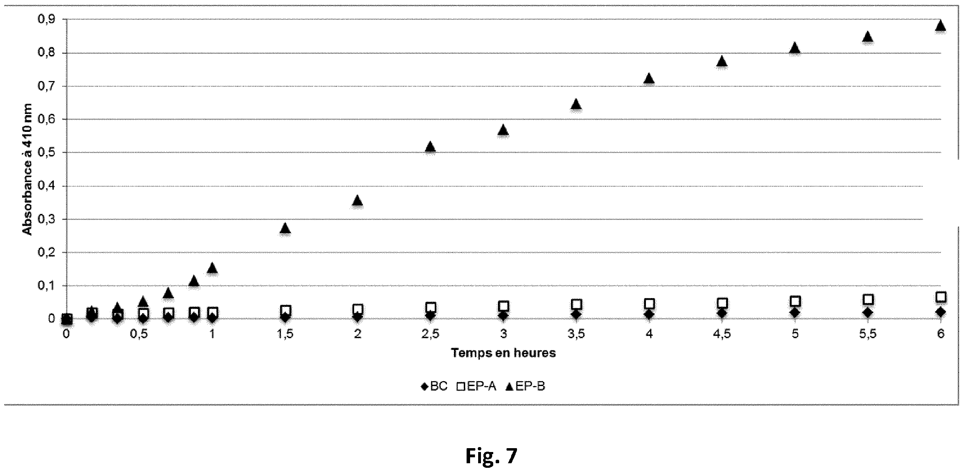

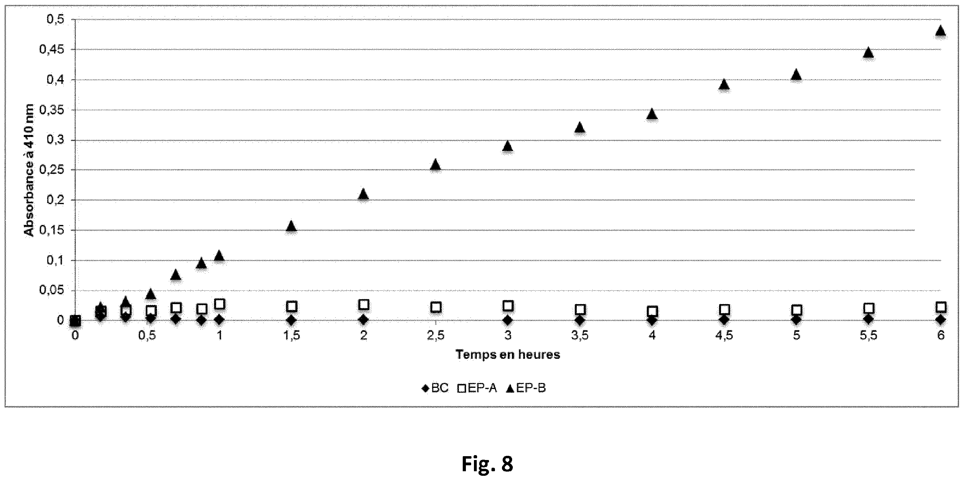

Ce test permet de conclure que les enzymes du malt d'orge ont non seulement un site de coupure différent entre-elles mais aussi différent de celui du Brewers Clarex (**Figures 7** **et** **8**).

### Test lyophilisation

Les inventeurs ont observé que l'activité enzymatique était préservée après lyophilisation.

Les deux tampons testés permettent de conserver l'activité, mais le tampon Tris/EDTA/Cystéine/mannitol/saccharose s'avère être plus efficace.

### Conclusions

Les inventeurs ont montré que deux fractions enzymatiques isolées du malt d'orge étaient capables de diminuer le trouble colloïdal de la bière et d'hydrolyser les prolamines des céréales (hordéines et gliadines). Ces deux fractions enzymatiques sont enrichies, respectivement en deux protéases EP-B et EP-A, des cystéines protéinases de l'orge. Ces deux enzymes sont très proches au niveau de leurs séquences en acides aminés (structure primaire) et leurs séquences ne présentent aucune identité avec celle de l'enzyme Brewers Clarex®. Enfin l'hydrolyse des prolamines (hordéines, gliadines) ainsi que de la β-lactoglobuline, de même que l'hydrolyse des peptides Z-Gly-Pro-pNA et Z-Phe-Arg-pNA, montrent que ces cystéines protéinases ont des spécificités (sites de clivage) différentes et aussi différentes de celle de l'enzyme Brewers Clarex®.

### Exemple 4 : Hydrolyse des gliadines par EP-A, EP-B et l'extrait brut de malt

Cet exemple montre que les enzymes EP-A et EP-B clivent les gliadines de manière différente, suggérant une synergie dans l'action des deux enzymes.

### - Matériel et méthodes :

Les gliadines sont solubilisées à 4mg/ml dans l'acide acétique 50mM puis diluées à 1mg/ml avec le tampon acétate de sodium 50mM à pH 5 et mélangées avec différents extraits. Le mélange de gliadines et de tampon acétate représente le témoin noté T, le mélange gliadines et extrait brut obtenu comme décrit dans l'exemple 3 est noté EB, le mélange de gliadines et d'EP-A obtenue comme décrit dans l'exemple 3 est noté EP-A, et le mélange de gliadines et d'EP-B obtenue comme décrit dans l'exemple 3 est noté EP-B. Les mélanges sont incubés 1 nuit à 37°C sous agitation.

Les quantités d'enzymes ajoutées ont été calculées afin d'obtenir un rapport enzyme substrat de 4%. Les échantillons sont incubés 18 heures à 37°C avant d'être analysés par chromatographie en phase liquide à haute performance (HPLC).

### - Analyse par HPLC en phase inverse :

Colonne phase inversée : Luna, C₁₈, 100A, 5µm, 250X4,6 mm (Phenomenex)
Eluant A : H2O, TFA 0,11%
Eluant B : Acétonitrile, TFA 0,09%
Débit 1ml/min ; détection spectrophotomètre à 214 et 280 nm. La mesure à 280 nm permet de détecter spécifiquement les protéines contenant des acides aminés aromatiques tel que le tryptophane. A 214 nm, le signal, bien que moins spécifique, est environ 10 fois plus important qu'à 280 nm.
L'élution a été effectuée par un gradient linéaire de 10 à 60% d'éluant B en 20 min.
Volume d'injection : 40µL. L'absorbance est enregistrée à 214 et 280 nm à l'aide d'un détecteur Waters 2487.

### Résultats

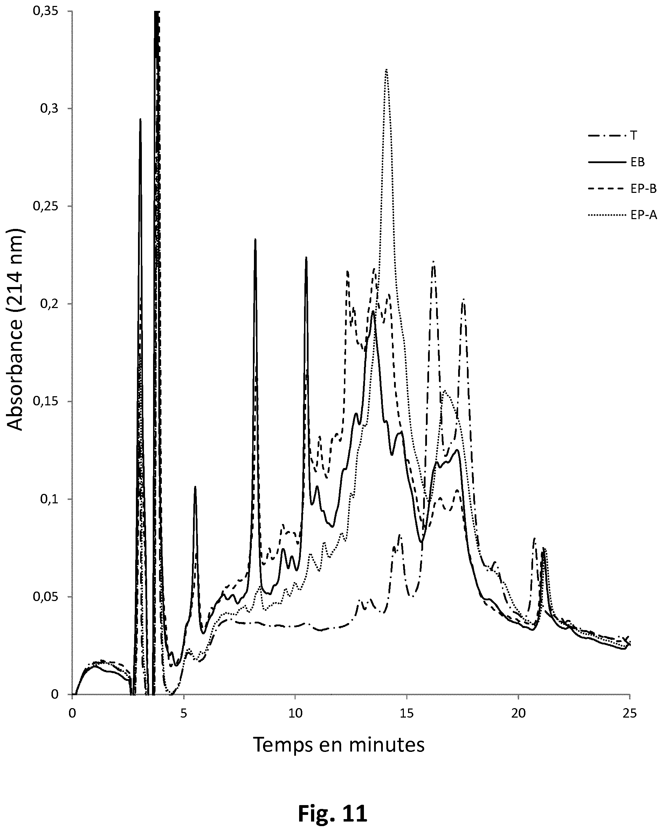

Les essais d'hydrolyse sont réalisés sur des gliadines totales. Les 2 pics principaux correspondant aux gliadines (temps d'élution 16,1 et 17,5 min) montrent que les gliadines sont partiellement hydrolysées par l'extrait brut (EB) et les 2 enzymes. Les pics apparaissant sur les chromatogrammes après hydrolyse par EP-B et EP-A sont différents, indiquant des sites de coupure différents (**Figure 11**). L'hydrolyse par EP-B est plus marquée que par EP-A, ce qui laisse supposer que de plus petites chaînes peptidiques sont libérées, tandis qu'EP-A permet une hydrolyse plus spécifique, marquée par un pic plus fort.

### Exemple 5 : Tests de fermentation en tubes EBC (type « KIRIN »)

Cet exemple montre l'effet d'augmentation du rendement de fermentation de l'extrait de malt selon l'invention.

### Protocoles de préparation des extraits de malt d'orge

### Broyage du malt

Du malt 2RP Pilsen a été stocké à 4°C, puis broyé sur broyeur ZM 200 avec une grille de 0.5mm.

### Extraction

Pour chaque extraction, 900 g de Malt sont utilisés avec 3.6 L de Tampon citrate. Elle dure 30 min à 7°C avec une agitation à 250rpm. Les extraits obtenus sont centrifugés durant 25 min à 8400 rpm et à une température de 4°C, avec une accélération et une décélération de 3. Le surnageant est récupéré, et grossièrement filtré sur des filtres Marcherey Nagel 614 1/4 de diamètre 320 mm.

### Désactivation pour l'Extrait Brut Dénaturé (EBD)

Le surnageant est chauffé sans agitation jusqu'à l'apparition des premiers bouillons, puis le surnageant est centrifugé durant 25 min à 8400 rpm à une température de 4°C et filtré grossièrement.

### Précipitation 15% (w/v) au sulfate d'ammonium (SA)

La précipitation par ajout de sulfate d'ammonium est effectuée dans les mêmes conditions que l'extraction, avec une agitation de 250 rpm et à 7°C. Le mélange est centrifugé durant 25 min à 8400 rpm et à 4°C. Le surnageant est récupéré et son volume est mesuré.

### Précipitation 65% (w/v) au SA

Le culot est récupéré dans 600 mL de Tampon citrate, puis filtré grossièrement avant filtration sur un filtre à 0.45µm. Le produit obtenu est conservé à 4°C en attendant le dessalage.

### Dessalage

Le dessalage est effectué sur un gel Sephadex G25 packé dans une colonne AxiChrom 70 (GE Healthcare). L'appareil utilisé est l'AKTA Prime Plus (GE Healthcare). Le dessalage se fait à température ambiante et l'échantillon est injecté avec un débit de 40 mL/min. L'élution se fait avec le tampon citrate 0.1M pH 4.3.

### Chromatographie par échange d'ions

Le gel utilisé est le gel Sepharose Fast Flow, équilibré avec du tampon citrate 50 mM pH5, la chromatographie se fait à température ambiante La cystéine endoprotéase se trouve dans la fraction éluée.

### Tests de fermentation

Un moût à 11° Plato est produit par brassage de type « congress EBC» : chauffage à 45°C pendant 30 minutes, suivi d'une montée en température à 70°C pendant 60 minutes, puis 210 g de malt sont versés pour 1260 mL d'eau.

Les différents ajouts (extraits de malt, contrôles...) se font avant ensemencement dans 770mL de moût, sauf le PVPP (40g /HL), qui est ajouté avant centrifugation.

Les modalités testées correspondent respectivement à:
- T- est un extrait brut de malt d'orge dénaturé par la chaleur
- Tₚᵥₚₚ: aucun ajout
- TBC est le témoin positif avec ajout de Brewers Clarex®
- EB est l'extrait brut de malt d'orge
- S15 est un extrait de malt traité par une solution de sulfate d'ammonium à 15%
- S65 est un extrait brut traité par une solution de sulfate d'ammonium à 15%, puis le surnageant est traité par une solution de sulfate d'ammonium à 50%
- 65CH est la modalité S65, purifiée sur colonne SP.

Les chiffres 1, 2 et 3 correspondent à 3 volumes d'ajouts différents testés : les volumes 1 vont de 15 à 30 mL, les volumes 2 sont de 22,5 et 30 mL, le volume 3 correspond à 30 ou 37,5 mL.

### Ensemencement:

- pour les fermentations : 720 mL avec de la levure sèche
- pour la mesure de l'Atténuation Limite Apparente: 50mL avec de la levure sèche

La fermentation / maturation est effectuée à une température de 13°C pendant environ 9 jours.

La levure est purgée, puis le moût est mis en garde au froid (environ 2°C, minimum 5 jours).

En fin de garde, les moûts fermentés sont centrifugés (Centrifugation à 4000 rpm, pendant 10 minutes) et le surnageant est analysé.

Afin de suivre la fermentation, du moût est prélevé, filtré sur Kieselguhr, puis une mesure de pourcentage en masse d'extrait sec du moût est effectuée. Cette mesure est exprimée en degrés Plato (noté °Plato) et mise en oeuvre grâce à un densimètre automatique Anton Paar DMA 35. Un suivi de la population de levures est également effectué.

### Résultats

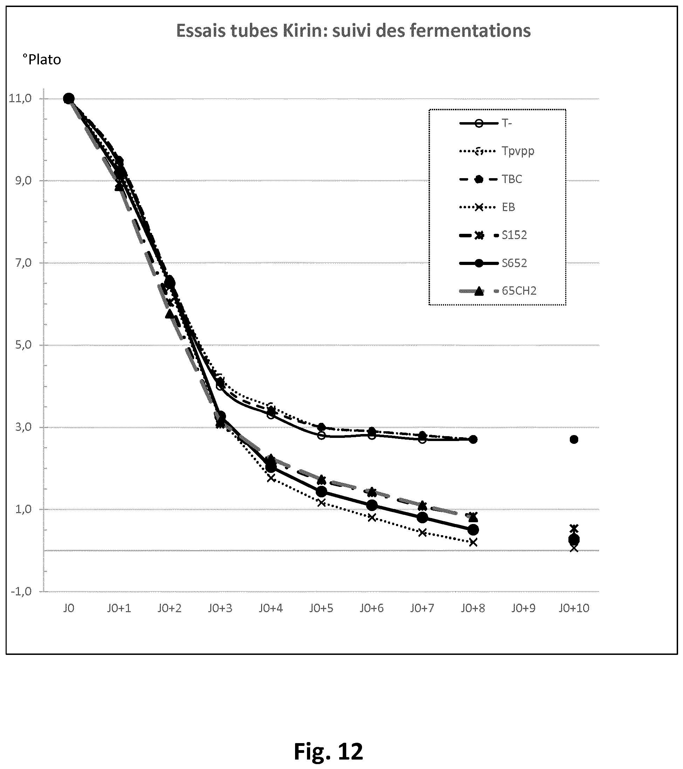

La fermentation est accélérée avec l'extrait de malt d'orge. A durée équivalente, dès J0+3, il reste moins de sucres fermentescibles en présence de malt d'orge qu'au cours de la fermentation du témoin constitué par le malt d'orge seul dénaturé par la chaleur, du témoin négatif et du témoin positif avec Brewers Clarex® (**Figure 12**).

## Revendications

1. Utilisation d'au moins une cystéine endoprotéase comprenant une séquence au moins 55% identique à la séquence SEQ ID NO : 1, à la séquence SEQ ID NO: 2, à la séquence SEQ ID NO : 3 et/ou à la séquence SEQ ID NO : 4, ou un fragment de celle-ci présentant une activité cystéine endoprotéase, pour diminuer le trouble d'une boisson, fermentée ou non, à base de céréales.

2. Utilisation selon la revendication 1, dans laquelle la au moins une cystéine endoprotéase est utilisée sous forme d'extrait de malt.

3. Utilisation d'un extrait de malt comprenant une activité endoprotéasique pour diminuer le trouble d'une boisson, fermentée ou non, à base de céréales.

4. Utilisation selon la revendication 3, dans laquelle l'extrait de malt comprend une activité cystéine endoprotéase A et/ou cystéine endoprotéase B.

5. Utilisation (i) d'au moins une cystéine endoprotéase telle que définie dans l'une quelconque des revendications 1 à 2 ou (ii) d'un extrait de malt tel que défini dans l'une quelconque des revendications 3 à 4, dans la préparation d'une boisson, fermentée ou non, à base de céréales.

6. Utilisation selon la revendication 1 à 5, dans laquelle la boisson, fermentée ou non, à base de céréales, est une bière.

7. Procédé de fabrication d'une boisson, fermentée ou non, à base de céréales, comprenant une étape d'ajout (i) d'au moins une cystéine endoprotéase telle que définie dans l'une quelconque des revendications 1 à 2 ou (ii) d'un extrait de malt tel que défini dans l'une quelconque des revendications 3 à 4, dans un moût ou un jus.

8. Procédé selon la revendication 7, dans lequel la boisson est une boisson fermentée, à base de céréales, et ladite au moins une cystéine endoprotéase ou ledit extrait de malt est ajouté au cours de l'étape de fermentation du procédé de fabrication de la boisson.

9. Procédé selon la revendication 8, comprenant une étape d'ajout (i) d'au moins une cystéine endoprotéase telle que définie dans l'une quelconque des revendications 1 à 2 ou (ii) d'un extrait de malt tel que défini dans l'une quelconque des revendications 3 à 4, dans un moût, ledit procédé comprenant les étapes suivantes :
a) une étape de maltage ou de fourniture du malt,
b) une étape de concassage ou broyage du malt obtenu à l'étape a),
c) une étape de mélange du malt broyé à l'étape b) avec de l'eau pour former une maische,
d) une étape de saccharification de la maische obtenue à l'étape c) pour former un moût,
e) une étape de filtration pour obtenir le moût primitif,
f) une étape d'ébullition comprenant une étape de houblonnage amérisante et aromatisante,
g) une étape de séparation tangentielle "whirlpool" ou par centrifugation pour séparer le trouble du moût,
h) une étape de refroidissement, d'ensemencement et d'oxygénation du moût,
i) une étape de fermentation, en présence de levures, au cours de laquelle, ladite au moins une cystéine endoprotéase ou ledit extrait de malt est ajouté,
j) une étape de garde du produit fermenté obtenu à l'étape g),
k) une étape de filtration et séparation des levures, et
l) une étape de conditionnement.

10. Boisson à base de céréales susceptible d'être obtenue par le procédé selon l'une quelconque des revendications 7 à 9.

11. Utilisation (i) d'au moins une cystéine endoprotéase telle que définie dans l'une quelconque des revendications 1 à 2 ou (ii) d'un extrait de malt tel que défini dans l'une quelconque des revendications 3 à 4, pour augmenter le rendement de fermentation lors de la fabrication d'une boisson fermentée à base de céréales.

## Patentansprüche

1. Verwendung mindestens einer Cysteinendoprotease, die eine Sequenz enthält, die mindestens 55 % identisch zu der Sequenz SEQ. ID NO: 1, zu der Sequenz SEQ ID NO: 2, zu der Sequenz SEQ ID NO: 3 und/oder zu der Sequenz SEQ ID NO: 4 ist oder ein Fragment derselben, das eine Cysteinendoprotease-Aktivität aufweist, um die Trübung eines fermentierten oder nicht fermentierten Getränks auf der Basis von Getreide zu verringern.

2. Verwendung nach Anspruch 1, bei dem mindestens eine Cysteinendoprotease in Form eines Malzextraktes verwendet wird.

3. Verwendung eines Malzextraktes, das eine endoproteasische Aktivität aufweist, um die Trübung eines fermentierten oder nicht fermentierten Getränks auf der Basis von Getreide zu verringern.

4. Verwendung nach Anspruch 3, bei der der Malzextrakt eine Cysteinendoprotease-Aktivität A und/oder Cysteinendoprotease B enthält.

5. Verwendung (i) mindestens einer Cysteinendoprotease, die in einem beliebigen der Ansprüche 1 bis 2 definiert ist, oder (ii) eines Malzextraktes, wie in einem beliebigen der Ansprüche 3 bis 4 definiert ist, in der Herstellung eines fermentierten oder nicht fermentierten Getränks auf der Basis von Getreide.

6. Verwendung nach Anspruch 1 bis 5, bei der das fermentierte oder nicht fermentierte Getränk auf der Basis von Getreide Bier ist.

7. Verfahren zur Herstellung eines fermentierten oder nicht fermentierten Getränks auf der Basis von Getreide, umfassend einen Schritt des Hinzufügens (i) einer Cysteinendoprotease, wie sie in einem beliebigen der Ansprüche 1 bis 2 definiert ist, oder (ii) eines Malzextraktes, wie er in einem beliebigen der Ansprüche 3 bis 4 definiert ist, zu einer Würze oder einem Saft.

8. Verfahren nach Anspruch 7, bei dem das Getränk ein fermentiertes Getränk auf der Basis von Getreide ist und mindestens eine Cysteinendoprotease oder der Malzextrakt während des Schrittes der Fermentierung des Verfahrens zur Herstellung des Getränks hinzugefügt wird.

9. Verfahren nach Anspruch 8, umfassend einen Schritt des Hinzufügens (i) einer Cysteinendoprotease, wie sie in einem beliebigen der Ansprüche 1 bis 2 definiert ist, oder (ii) eines Malzextraktes, wie er in einem beliebigen der Ansprüche 3 bis 4 definiert ist, zu einer Würze, wobei das Verfahren die folgenden Schritte umfasst:
a) einen Schritt des Mälzens oder des Lieferns des Malzes,
b) einen Schritt des Zerkleinerns oder Mahlens des in Schritt a) erhaltenen Malzes,
c) einen Schritt des Mischens des in Schritt b) gemahlenen Malzes mit Wasser, um eine Maische zu bilden,
d) einen Schritt der Verzuckerung der in Schritt c) erhaltenen Maische, um eine Würze zu bilden,
e) einen Schritt des Filterns, um die Stammwürze zu erhalten,
f) einen Schritt des Siedens, der einen Schritt der Zugabe von bitterem und aromatisierendem Hopfen enthält,
g) einen Schritt des tangentialen Trennens "Whirlpool" oder des Trennens durch Zentrifugieren, um die Trübabscheidung der Würze durchzuführen,
h) einen Schritt des Abkühlens, Impfens und der Sauerstoffanreicherung der Würze,
i) einen Schritt des Gärens in Anwesenheit von Hefen, während dem die mindestens eine Cysteinendoprotease oder der Malzextrakt hinzugefügt wird,
j) einen Schritt des Aufbewahrens des im Schritt g) erhaltenen fermentierten Produktes,
k) einen Schritt des Filterns und Trennens der Hefen,
l) einen Schritt der Nachgärung.

10. Getränk auf Basis von Getreide, das geeignet ist, durch das Verfahren nach einem beliebigen der Ansprüche 7 bis 9 erhalten zu werden.

11. Verwendung (i) mindestens einer Cysteinendoprotease, wie sie in einem beliebigen der Ansprüche 1 bis 2 definiert ist, oder (ii) eines Malzextraktes, wie er in einem beliebigen der Ansprüche 3 bis 4 definiert ist, um die Ausbeute der Fermentierung bei der Herstellung eines fermentierten Getränkes auf der Basis von Getreide zu erhöhen.

## Claims

1. Use of at least one cysteine endoprotease comprising a sequence that is at least 55% identical to the sequence SEQ ID NO: 1, sequence SEQ ID NO: 2, sequence SEQ ID NO: 3 and/or sequence SEQ ID NO: 4, or a fragment thereof having cysteine endoprotease activity, for decreasing the cloudiness of a fermented or non-fermented cereal-based beverage.

2. Use according to claim 1, wherein the at least one cysteine endoprotease is used in the form of malt extract.

3. Use of a malt extract comprising an endoprotease activity for reducing the cloudiness of a cereal-based beverage, fermented or not.

4. Use according to claim 3, wherein the malt extract comprises cysteine endoprotease A and/or cysteine endoprotease B activity.

5. Use of (i) at least one cysteine endoprotease as defined in any one of claims 1 to 2 or (ii) a malt extract as defined in any one of claims 3 to 4, in the preparation of a cereal-based beverage, fermented or not.

6. Use according to claim 1 to 5, wherein the fermented or non-fermented cereal-based beverage is a beer.

7. Method for producing a cereal-based fermented or non-fermented beverage, comprising a step of adding (i) at least one cysteine endoprotease as defined in any one of claims 1 to 2, or (ii) a malt extract as defined in any one of claims 3 to 4, in a wort or a juice.

8. Method according to claim 7, wherein the beverage is a cereal-based fermented beverage, and wherein said at least one cysteine endoprotease or said malt extract is added during the fermentation step of the method of production of the beverage.

9. Method according to claim 8, comprising a step of adding (i) at least one cysteine endoprotease as defined in any one of claims 1 to 2 or (ii) a malt extract as defined in any one of claims 3 to 7, in a wort, said method comprising the following steps:
a) a step of malting or of providing malt,
b) a step of crushing or milling the malt obtained in step a),
c) a step of mixing the malt milled in step b) with water to form a mash,
d) a step of saccharifying the mash obtained in step c) to form a wort,
e) a filtration step for obtaining the primary wort,
f) a boiling step comprising a bittering and flavoring hopping step,
g) a "whirlpool" tangential separation or centrifugation step to separate the cloudiness from the wort,
h) a step of cooling, seeding and oxygenating the wort,
i) a fermentation step, in the presence of yeasts, during which step said at least one cysteine endoprotease or said malt extract is added,
j) a step of storing the fermented product obtained in step g),
k) a step of filtration and separation of the yeasts, and
l) a conditioning step.

10. Cereal-based beverage obtainable by the method according to any one of claims 7 to 9.

11. Use of (i) at least one cysteine endoprotease as defined in any one of claims 1 to 2, or (ii) a malt extract as defined in any one of claims 3 to 4, to increase the fermentation yield during the production of a fermented cereal-based beverage.


### SEQUENCE LISTING

<110> Malteries Soufflet
   INRA
<120> Utilisation de cystéine endoprotéase pour diminuer le trouble de boissons
<130> BET16P0699
<150> FR15/53869
   <151> 2015-04-29
<160> 8
<170> PatentIn version 3.5
<210> 1
   <211> 365
   <212> PRT
   <213> Hordeum vulgare
<400> 1
   Met Trp Arg Cys Ile Leu Leu Ser Ala Val Val Val Ala Leu Ala Leu
   1               5                   10                  15
   Ala Pro Ala Pro Ala Leu Gly Val Pro Phe Thr Glu Lys Asp Leu Ala
               20                  25                  30
   Ser Glu Glu Ser Leu Arg Gly Leu Tyr Glu Arg Trp Arg Ser His Tyr
           35                  40                  45
   Thr Val Ser Arg Arg Gly Leu Gly Ala Asp Ala Glu Glu Arg Arg Phe
       50                  55                  60
   Asn Val Phe Lys Glu Asn Ala Arg Tyr Val His Glu Gly Asn Lys Arg
   65                  70                  75                  80
   Asp Arg Pro Phe Arg Leu Ala Leu Asn Lys Phe Ala Asp Met Thr Thr
                   85                  90                  95
   Asp Glu Phe Arg Arg Thr Tyr Ala Gly Ser Arg Val Arg His His Leu
               100                 105                 110
   Ser Leu Ser Gly Gly Arg Arg Gly Asp Gly Gly Phe Arg Tyr Ala Asp
           115                 120                 125
   Ala Asp Asn Leu Pro Pro Ala Val Asp Trp Arg Gln Lys Gly Ala Val
       130                 135                 140
   Thr Ala Ile Lys Asp Gln Gly Gln Cys Gly Ser Cys Trp Ala Phe Ser
   145                 150                 155                 160
   Thr Ile Val Ala Val Glu Gly Ile Asn Lys Ile Arg Thr Gly Lys Leu
                   165                 170                 175
   Val Ser Leu Ser Glu Gln Glu Leu Met Asp Cys Asp Asn Val Asn Asn
               180                 185                 190
   Gln Gly Cys Glu Gly Gly Leu Met Asp Tyr Ala Phe Gln Phe Ile Gln
           195                 200                 205
   Lys Asn Gly Ile Thr Thr Glu Ser Asn Tyr Pro Tyr Gln Gly Glu Gln
       210                 215                 220
   Gly Ser Cys Asp Gln Ala Lys Glu Asn Ala Gln Ala Val Thr Ile Asp
   225                 230                 235                 240
   Gly Tyr Glu Asp Val Pro Ala Asn Asp Glu Ser Ala Leu Gln Lys Ala
                   245                 250                 255
   Val Ala Gly Gln Pro Val Ser Val Ala Ile Asp Ala Ser Gly Gln Asp
               260                 265                 270
   Phe Gln Phe Tyr Ser Glu Gly Val Phe Thr Gly Glu Cys Ser Thr Asp
           275                 280                 285
   Leu Asp His Gly Val Ala Ala Val Gly Tyr Gly Ala Thr Arg Asp Gly
       290                 295                 300
   Thr Lys Tyr Trp Ile Val Lys Asn Ser Trp Gly Glu Asp Trp Gly Glu
   305                 310                 315                 320
   Lys Gly Tyr Ile Arg Met Gln Arg Gly Val Ser Gln Thr Glu Gly Leu
                   325                 330                 335
   Cys Gly Ile Ala Met Gln Ala Ser Tyr Pro Thr Lys Ser Ala Pro His
               340                 345                 350
   Ala Ser Thr Val Arg Glu Gly Ser His Thr Asp Glu Leu
           355                 360                 365
<210> 2
   <211> 365
   <212> PRT
   <213> Hordeum vulgare
<400> 2
   Met Trp Arg Cys Ile Leu Leu Ser Ala Val Val Val Ala Leu Ala Leu
   1               5                   10                  15
   Ala Pro Ala Pro Ala Leu Gly Val Pro Phe Thr Glu Lys Asp Leu Ala
               20                  25                  30
   Ser Glu Glu Ser Leu Arg Gly Leu Tyr Glu Arg Trp Arg Ser His Tyr
           35                  40                  45
   Thr Val Ser Arg Arg Gly Leu Gly Ala Asp Ala Glu Glu Arg Arg Phe
       50                  55                  60
   Asn Val Phe Lys Gln Asn Ala Arg Tyr Val His Glu Gly Asn Lys Arg
   65                  70                  75                  80
   Asp Met Pro Phe Arg Leu Ala Leu Asn Lys Phe Ala Asp Met Thr Thr
                   85                  90                  95
   Asp Glu Phe Arg Arg Thr Tyr Ala Gly Ser Arg Val Arg His His Leu
               100                 105                 110
   Ser Leu Ser Gly Gly Arg Arg Gly Asp Gly Gly Phe Arg Tyr Gly Asp
           115                 120                 125
   Ala Asp Asn Leu Pro Pro Ala Val Asp Trp Arg Gln Lys Gly Ala Val
       130                 135                 140
   Thr Ala Ile Lys Asp Gln Gly Gln Cys Gly Ser Cys Trp Ala Phe Ser
   145                 150                 155                 160
   Thr Ile Val Ala Val Glu Gly Ile Asn Lys Ile Arg Thr Gly Lys Leu
                   165                 170                 175
   Val Ser Leu Ser Glu Gln Glu Leu Met Asp Cys Asp Asn Val Asn Asn
               180                 185                 190
   Gln Gly Cys Asp Gly Gly Leu Met Asp Tyr Ala Phe Gln Phe Ile Gln
           195                 200                 205
   Lys Asn Gly Ile Thr Thr Glu Ser Asn Tyr Pro Tyr Gln Gly Glu Gln
       210                 215                 220
   Gly Ser Cys Asp Gln Ala Lys Glu Asn Ala Gln Ala Val Thr Ile Asp
   225                 230                 235                 240
   Gly Tyr Glu Asp Val Pro Ala Asn Asp Glu Ser Ala Leu Gln Lys Ala
                   245                 250                 255
   Val Ala Gly Gln Pro Val Ser Val Ala Ile Asp Ala Ser Gly Gln Asp
               260                 265                 270
   Phe Gln Phe Tyr Ser Glu Gly Val Phe Thr Gly Glu Cys Ser Thr Asp
           275                 280                 285
   Leu Asp His Gly Val Ala Ala Val Gly Tyr Gly Ala Thr Arg Asp Gly
       290                 295                 300
   Thr Lys Tyr Trp Ile Val Lys Asn Ser Trp Gly Glu Asp Trp Gly Glu
   305                 310                 315                 320
   Lys Gly Tyr Ile Arg Met Gln Arg Gly Val Ser Gln Thr Glu Gly Leu
                   325                 330                 335
   Cys Gly Ile Ala Met Gln Ala Ser Tyr Pro Thr Lys Ser Ala Pro His
               340                 345                 350
   Ala Ser Thr Val Arg Glu Glu Ser His Thr Asp Glu Leu
           355                 360                 365
<210> 3
   <211> 371
   <212> PRT
   <213> Hordeum vulgare
<400> 3
   Met Gly Leu Leu Ser Lys Lys Leu Leu Val Ala Ser Met Val Ala Ala
   1               5                   10                  15
   Val Leu Ala Val Ala Ala Val Glu Leu Cys Ser Ala Ile Pro Met Glu
               20                  25                  30
   Asp Lys Asp Leu Glu Ser Glu Glu Ala Leu Trp Asp Leu Tyr Glu Arg
           35                  40                  45
   Trp Gln Ser Ala His Arg Val Arg Arg His His Ala Glu Lys His Arg
       50                  55                  60
   Arg Phe Gly Thr Phe Lys Ser Asn Ala His Phe Ile His Ser His Asn
   65                  70                  75                  80
   Lys Arg Gly Asp His Pro Tyr Arg Leu His Leu Asn Arg Phe Gly Asp
                   85                  90                  95
   Met Asp Gln Ala Glu Phe Arg Ala Thr Phe Val Gly Asp Leu Arg Arg
               100                 105                 110
   Asp Thr Pro Ala Lys Pro Pro Ser Val Pro Gly Phe Met Tyr Ala Ala
           115                 120                 125
   Leu Asn Val Ser Asp Leu Pro Pro Ser Val Asp Trp Arg Gln Lys Gly
       130                 135                 140
   Ala Val Thr Gly Val Lys Asp Gln Gly Lys Cys Gly Ser Cys Trp Ala
   145                 150                 155                 160
   Phe Ser Thr Val Val Ser Val Glu Gly Ile Asn Ala Ile Arg Thr Gly
                   165                 170                 175
   Ser Leu Val Ser Leu Ser Glu Gln Glu Leu Ile Asp Cys Asp Thr Ala
               180                 185                 190
   Asp Asn Asp Gly Cys Gln Gly Gly Leu Met Asp Asn Ala Phe Glu Tyr
           195                 200                 205
   Ile Lys Asn Asn Gly Gly Leu Ile Thr Glu Ala Ala Tyr Pro Tyr Arg
       210                 215                 220
   Ala Ala Arg Gly Thr Cys Asn Val Ala Arg Ala Ala Gln Asn Ser Pro
   225                 230                 235                 240
   Val Val Val His Ile Asp Gly His Gln Asp Val Pro Ala Asn Ser Glu
                   245                 250                 255
   Glu Asp Leu Ala Arg Ala Val Ala Asn Gln Pro Val Ser Val Ala Val
               260                 265                 270
   Glu Ala Ser Gly Lys Ala Phe Met Phe Tyr Ser Glu Gly Val Phe Thr
           275                 280                 285
   Gly Asp Cys Gly Thr Glu Leu Asp His Gly Val Ala Val Val Gly Tyr
       290                 295                 300
   Gly Val Ala Glu Asp Gly Lys Ala Tyr Trp Thr Val Lys Asn Ser Trp
   305                 310                 315                 320
   Gly Pro Ser Trp Gly Glu Gln Gly Tyr Ile Arg Val Glu Lys Asp Ser
                   325                 330                 335
   Gly Ala Ser Gly Gly Leu Cys Gly Ile Ala Met Glu Ala Ser Tyr Pro
               340                 345                 350
   Val Lys Thr Tyr Asn Lys Pro Met Pro Arg Arg Ala Leu Gly Ala Trp
           355                 360                 365
   Glu Ser Gln
   370
<210> 4
   <211> 373
   <212> PRT
   <213> Hordeum vulgare
<400> 4
   Met Gly Leu Leu Ser Lys Lys Leu Leu Val Ala Ser Met Val Ala Ala
   1               5                   10                  15
   Val Leu Ala Val Ala Ala Val Glu Leu Cys Ser Ala Ile Pro Met Glu
               20                  25                  30
   Asp Lys Asp Leu Glu Ser Glu Glu Ala Leu Trp Asp Leu Tyr Glu Arg
           35                  40                  45
   Trp Gln Ser Ala His Arg Val Arg Arg His His Ala Glu Lys His Arg
       50                  55                  60
   Arg Phe Gly Thr Phe Lys Ser Asn Ala His Phe Ile His Ser His Asn
   65                  70                  75                  80
   Lys Arg Gly Asp His Pro Tyr Arg Leu His Leu Asn Arg Phe Gly Asp
                   85                  90                  95
   Met Asp Gln Ala Glu Phe Arg Ala Thr Phe Val Gly Asp Leu Arg Arg
               100                 105                 110
   Asp Thr Pro Ser Lys Pro Pro Ser Val Pro Gly Phe Met Tyr Ala Ala
           115                 120                 125
   Leu Asn Val Ser Asp Leu Pro Pro Ser Val Asp Trp Arg Gln Lys Gly
       130                 135                 140
   Ala Val Thr Gly Val Lys Asp Gln Gly Lys Cys Gly Ser Cys Trp Ala
   145                 150                 155                 160
   Phe Ser Thr Val Val Ser Val Glu Gly Ile Asn Ala Ile Arg Thr Gly
                   165                 170                 175
   Ser Leu Val Ser Leu Ser Glu Gln Glu Leu Ile Asp Cys Asp Thr Ala
               180                 185                 190
   Asp Asn Asp Gly Cys Gln Gly Gly Leu Met Asp Asn Ala Phe Glu Tyr
           195                 200                 205
   Ile Lys Asn Asn Gly Gly Leu Ile Thr Glu Ala Ala Tyr Pro Tyr Arg
   210 215 220
   Ala Ala Arg Gly Thr Cys Asn Val Ala Arg Ala Ala Gln Asn Ser Pro
   225 230 235 240
   Val Val Val His Ile Asp Gly His Gln Asp Val Pro Ala Asn Ser Glu
   245 250 255
   Glu Asp Leu Ala Arg Ala Val Ala Asn Gln Pro Val Ser Val Ala Val
   260 265 270
   Glu Ala Ser Gly Lys Ala Phe Met Phe Tyr Ser Glu Gly Val Phe Thr
   275 280 285
   Gly Glu Cys Gly Thr Glu Leu Asp His Gly Val Ala Val Val Gly Tyr
   290 295 300
   Gly Val Ala Glu Asp Gly Lys Ala Tyr Trp Thr Val Lys Asn Ser Trp
   305 310 315 320
   Gly Pro Ser Trp Gly Glu Gln Gly Tyr Ile Arg Val Glu Lys Asp Ser
   325 330 335
   Gly Ala Ser Gly Gly Leu Cys Gly Ile Ala Met Glu Ala Ser Tyr Pro
   340 345 350
   Val Lys Thr Tyr Ser Lys Pro Lys Pro Thr Pro Arg Arg Ala Leu Gly
   355 360 365
   Ala Arg Glu Ser Leu
   370
<210> 5
   <211> 238
   <212> PRT
   <213> Hordeum vulgare
<400> 5
   Asp Ala Asp Asn Leu Pro Pro Ala Val Asp Trp Arg Gln Lys Gly Ala
   1               5                   10                  15
   Val Thr Ala Ile Lys Asp Gln Gly Gln Cys Gly Ser Cys Trp Ala Phe
               20                  25                  30
   Ser Thr Ile Val Ala Val Glu Gly Ile Asn Lys Ile Arg Thr Gly Lys
           35                  40                  45
   Leu Val Ser Leu Ser Glu Gln Glu Leu Met Asp Cys Asp Asn Val Asn
       50                  55                  60
   Asn Gln Gly Cys Glu Gly Gly Leu Met Asp Tyr Ala Phe Gln Phe Ile
   65                  70                  75                  80
   Gln Lys Asn Gly Ile Thr Thr Glu Ser Asn Tyr Pro Tyr Gln Gly Glu
                   85                  90                  95
   Gln Gly Ser Cys Asp Gln Ala Lys Glu Asn Ala Gln Ala Val Thr Ile
               100                 105                 110
   Asp Gly Tyr Glu Asp Val Pro Ala Asn Asp Glu Ser Ala Leu Gln Lys
           115                 120                 125
   Ala Val Ala Gly Gln Pro Val Ser Val Ala Ile Asp Ala Ser Gly Gln
       130                 135                 140
   Asp Phe Gln Phe Tyr Ser Glu Gly Val Phe Thr Gly Glu Cys Ser Thr
   145                 150                 155                 160
   Asp Leu Asp His Gly Val Ala Ala Val Gly Tyr Gly Ala Thr Arg Asp
                   165                 170                 175
   Gly Thr Lys Tyr Trp Ile Val Lys Asn Ser Trp Gly Glu Asp Trp Gly
               180                 185                 190
   Glu Lys Gly Tyr Ile Arg Met Gln Arg Gly Val Ser Gln Thr Glu Gly
           195                 200                 205
   Leu Cys Gly Ile Ala Met Gln Ala Ser Tyr Pro Thr Lys Ser Ala Pro
       210                 215                 220
   His Ala Ser Thr Val Arg Glu Gly Ser His Thr Asp Glu Leu
   225                 230                 235
<210> 6
   <211> 238
   <212> PRT
   <213> Hordeum vulgare
<400> 6
   Asp Ala Asp Asn Leu Pro Pro Ala Val Asp Trp Arg Gln Lys Gly Ala
   1               5                   10                  15
   Val Thr Ala Ile Lys Asp Gln Gly Gln Cys Gly Ser Cys Trp Ala Phe
               20                  25                  30
   Ser Thr Ile Val Ala Val Glu Gly Ile Asn Lys Ile Arg Thr Gly Lys
               35                  40                  45
   Leu Val Ser Leu Ser Glu Gln Glu Leu Met Asp Cys Asp Asn Val Asn
       50                  55                  60
   Asn Gln Gly Cys Asp Gly Gly Leu Met Asp Tyr Ala Phe Gln Phe Ile
   65                  70                  75                  80
   Gln Lys Asn Gly Ile Thr Thr Glu Ser Asn Tyr Pro Tyr Gln Gly Glu
                   85                  90                  95
   Gln Gly Ser Cys Asp Gln Ala Lys Glu Asn Ala Gln Ala Val Thr Ile
               100                 105                 110
   Asp Gly Tyr Glu Asp Val Pro Ala Asn Asp Glu Ser Ala Leu Gln Lys
           115                 120                 125
   Ala Val Ala Gly Gln Pro Val Ser Val Ala Ile Asp Ala Ser Gly Gln
       130                 135                 140
   Asp Phe Gln Phe Tyr Ser Glu Gly Val Phe Thr Gly Glu Cys Ser Thr
   145                 150                 155                 160
   Asp Leu Asp His Gly Val Ala Ala Val Gly Tyr Gly Ala Thr Arg Asp
                   165                 170                 175
   Gly Thr Lys Tyr Trp Ile Val Lys Asn Ser Trp Gly Glu Asp Trp Gly
               180                 185                 190
   Glu Lys Gly Tyr Ile Arg Met Gln Arg Gly Val Ser Gln Thr Glu Gly
           195                 200                 205
   Leu Cys Gly Ile Ala Met Gln Ala Ser Tyr Pro Thr Lys Ser Ala Pro
       210                 215                 220
   His Ala Ser Thr Val Arg Glu Glu Ser His Thr Asp Glu Leu
   225                 230                 235
<210> 7
   <211> 241
   <212> PRT
   <213> Hordeum vulgare
<400> 7
   Val Ser Asp Leu Pro Pro Ser Val Asp Trp Arg Gln Lys Gly Ala Val
   1               5                   10                  15
   Thr Gly Val Lys Asp Gln Gly Lys Cys Gly Ser Cys Trp Ala Phe Ser
               20                  25                  30
   Thr Val Val Ser Val Glu Gly Ile Asn Ala Ile Arg Thr Gly Ser Leu
           35                  40                  45
   Val Ser Leu Ser Glu Gln Glu Leu Ile Asp Cys Asp Thr Ala Asp Asn
       50                  55                  60
   Asp Gly Cys Gln Gly Gly Leu Met Asp Asn Ala Phe Glu Tyr Ile Lys
   65                  70                  75                  80
   Asn Asn Gly Gly Leu Ile Thr Glu Ala Ala Tyr Pro Tyr Arg Ala Ala
                   85                  90                  95
   Arg Gly Thr Cys Asn Val Ala Arg Ala Ala Gln Asn Ser Pro Val Val
               100                 105                 110
   Val His Ile Asp Gly His Gln Asp Val Pro Ala Asn Ser Glu Glu Asp
           115                 120                 125
   Leu Ala Arg Ala Val Ala Asn Gln Pro Val Ser Val Ala Val Glu Ala
       130                 135                 140
   Ser Gly Lys Ala Phe Met Phe Tyr Ser Glu Gly Val Phe Thr Gly Asp
   145                 150                 155                 160
   Cys Gly Thr Glu Leu Asp His Gly Val Ala Val Val Gly Tyr Gly Val
                   165                 170                 175
   Ala Glu Asp Gly Lys Ala Tyr Trp Thr Val Lys Asn Ser Trp Gly Pro
               180                 185                 190
   Ser Trp Gly Glu Gln Gly Tyr Ile Arg Val Glu Lys Asp Ser Gly Ala
           195                 200                 205
   Ser Gly Gly Leu Cys Gly Ile Ala Met Glu Ala Ser Tyr Pro Val Lys
       210                 215                 220
   Thr Tyr Asn Lys Pro Met Pro Arg Arg Ala Leu Gly Ala Trp Glu Ser
   225                 230                 235                 240
   Gln
<210> 8
   <211> 243
   <212> PRT
   <213> Hordeum vulgare
<400> 8
   Val Ser Asp Leu Pro Pro Ser Val Asp Trp Arg Gln Lys Gly Ala Val
   1               5                   10                  15
   Thr Gly Val Lys Asp Gln Gly Lys Cys Gly Ser Cys Trp Ala Phe Ser
               20                  25                  30
   Thr Val Val Ser Val Glu Gly Ile Asn Ala Ile Arg Thr Gly Ser Leu
           35                  40                  45
   Val Ser Leu Ser Glu Gln Glu Leu Ile Asp Cys Asp Thr Ala Asp Asn
       50                  55                  60
   Asp Gly Cys Gln Gly Gly Leu Met Asp Asn Ala Phe Glu Tyr Ile Lys
   65                  70                  75                  80
   Asn Asn Gly Gly Leu Ile Thr Glu Ala Ala Tyr Pro Tyr Arg Ala Ala
                   85                  90                  95
   Arg Gly Thr Cys Asn Val Ala Arg Ala Ala Gln Asn Ser Pro Val Val
               100                 105                 110
   Val His Ile Asp Gly His Gln Asp Val Pro Ala Asn Ser Glu Glu Asp
           115                 120                 125
   Leu Ala Arg Ala Val Ala Asn Gln Pro Val Ser Val Ala Val Glu Ala
       130                 135                 140
   Ser Gly Lys Ala Phe Met Phe Tyr Ser Glu Gly Val Phe Thr Gly Glu
   145                 150                 155                 160
   Cys Gly Thr Glu Leu Asp His Gly Val Ala Val Val Gly Tyr Gly Val
                   165                 170                 175
   Ala Glu Asp Gly Lys Ala Tyr Trp Thr Val Lys Asn Ser Trp Gly Pro
               180                 185                 190
   Ser Trp Gly Glu Gln Gly Tyr Ile Arg Val Glu Lys Asp Ser Gly Ala
           195                 200                 205
   Ser Gly Gly Leu Cys Gly Ile Ala Met Glu Ala Ser Tyr Pro Val Lys
       210                 215                 220
   Thr Tyr Ser Lys Pro Lys Pro Thr Pro Arg Arg Ala Leu Gly Ala Arg
   225                 230                 235                 240
   Glu Ser Leu